# FASCICULE DE BREVET EUROPEEN

(11) **EP 2 310 511 B1**
(45) Date de publication et mention de la délivrance du brevet: **14.01.2015**
(21) Numéro de dépôt: 09784259.5
(22) Date de dépôt: 09.07.2009
(51) Int. Cl.: C12N 15/70, C12P 21/00

(54) **VECTEUR AUTO REPLICATIF DEPOURVU DE GENE DE RESISTANCE A UN ANTIBIOTIQUE**
SELBSTREPLIZIERENDER VEKTOR OHNE ANTIBIOTIKARESISTENZGEN
SELF-REPLICATING VECTOR LACKING AN ANTIBIOTIC-RESISTANCE GENE

(30) Priorité: 18.07.2008 FR 0854887; 10.10.2008 US 104524 P
(43) Date de publication de la demande: 20.04.2011
(73) Titulaire: Sanofi Pasteur, 69367 Lyon Cedex 07 (FR)
(72) Inventeur: SODOYER, Régis, F-69290 Saint-Genis Les Ollières (FR); SPECK, Denis, F-69110 Sainte Foy Les Lyon (FR); PEUBEZ, Isabelle, F-69210 Saint-Pierre La Palud (FR); CHAUDET, Nicolas, F-69510 Soucieu en Jarrest (FR)
(86) Numéro de dépôt international: PCT/FR2009/000851
(87) Numéro de publication internationale: WO 2010/007246

(56) Documents cités:
- SZPIRER CÉDRIC Y ET AL: "Separate-component-stabilization system for protein and DNA production without the use of antibiotics" BIOTECHNIQUES, INFORMA LIFE SCIENCES PUBLISHING, WESTBOROUGH, MA, US, vol. 38, no. 5, 1 mai 2005 (2005-05-01), pages 775-781, XP001538926 ISSN: 0736-6205 cité dans la demande
- BALDING ET AL: "A mutational analysis of the ColE1-encoded cell cycle regulator Rcd confirms its role in plasmid stability" PLASMID, NEW YORK,NY, US, vol. 56, no. 1, 1 juillet 2006 (2006-07-01), pages 68-73, XP005439075 ISSN: 0147-619X
- SHARPE MICHAELA E ET AL: "Analysis of the ColE1 stability determinant Rcd" MICROBIOLOGY (READING), vol. 145, no. 8, août 1999 (1999-08), pages 2135-2144, XP009110004 ISSN: 1350-0872
- WEGERER ANGELIKA ET AL: "Optimization of an E. coli L-rhamnose-inducible expression vector: test of various genetic module combinations." BMC BIOTECHNOLOGY 2008, vol. 8, 14 janvier 2008 (2008-01-14), page 2, XP002507992 ISSN: 1472-6750

## Description

La présente invention a pour objet un nouveau vecteur et son utilisation pour la production d'une protéine hétérologue ou d'un gène d'intérêt, utilisable par exemple dans le cadre d'un programme d'immunisation ou de thérapie génique.

Les vecteurs comprennent au moins un marqueur de sélection dont la présence est nécessaire durant leur procédé de construction ainsi que durant la phase de culture cellulaire qui conduit à leur amplification et éventuellement à la production d'une protéine d'intérêt.

Les marqueurs de sélection utilisés classiquement sont les gènes de résistance aux antibiotiques.

Les risques potentiellement associés à l'utilisation de ces gènes de résistance, tels que la dissémination dans l'environnement ou le transfert du gène à une souche pathogène, ont conduit les autorités de santé à limiter l'utilisation de ces derniers. La présence d'un gène de résistance à un antibiotique est considérée aujourd'hui comme un inconvénient majeur pour une utilisation chez l'homme.

Plusieurs solutions alternatives ont donc été proposées. On peut citer par exemple les systèmes de sélection basés sur la complémentation d'un gène essentiel. Ces systèmes présentent tous l'inconvénient de nécessiter la construction d'une souche particulière déficiente dans ledit gène essentiel et l'utilisation obligatoire de milieux définis ne contenant pas le produit du gène essentiel.

La présente invention a pour objectif de fournir un nouveau vecteur utilisable à échelle industrielle qui présente le double avantage de conduire à un rendement d'expression élevé et ce en l'absence de toute utilisation d'antibiotique et qui peut donc d'être utilisé pour des grands volumes (ex 1000-10 000 litres)

Ce système est donc particulièrement avantageux dans le cadre d'une production industrielle de composants utilisables chez l'homme.

La présente invention fournit donc, un vecteur auto-réplicatif dépourvu de gène de résistance à un antibiotique comprenant:
- une séquence codant pour la protéine ccdA liée de façon fonctionnelle à un premier promoteur,
- la séquence du locus Cer et
- une séquence hétérologue, liée de façon fonctionnelle à un deuxième promoteur.

Selon un mode de réalisation particulier le premier promoteur est le promoteur constitutif mob.

Selon un autre mode de réalisation, le deuxième promoteur est un promoteur inductible, en particulier le deuxième promoteur est le promoteur T7.

Selon un mode de réalisation particulier, la séquence hétérologue code pour un antigène vaccinal.

Selon un autre mode de réalisation particulier, la séquence hétérologue correspond à une séquence utilisable dans le cadre d'une thérapie génique. Selon encore un autre mode de réalisation, la séquence hétérologue code pour une enzyme.

Selon un autre aspect, la présente invention concerne une cellule procaryote exprimant la protéine ccdB comprenant un vecteur tel que défini ci-dessus.

Selon un aspect particulier, ladite cellule procaryote est une cellule d'E.coli.

Selon un autre aspect, la présente invention concerne un procédé de production d'une protéine hétérologue comprenant les étapes de :
(a) ensemencement d'un milieu de culture approprié avec des cellules procaryotes exprimant la protéine ccdB et contenant un vecteur tel que défini ci-dessus,
(b) culture en fermenteur de la cellule ainsi transformée en l'absence d'antibiotique et
(c) récupération de la protéine hétérologue produite au cours de l'étape (b) à partir du surnageant ou du culot cellulaire.

Selon un mode de réalisation particulier, la présente invention concerne un procédé de production de rEPA.

Selon un autre aspect, la présente invention concerne un procédé de production d'un vecteur auto réplicatif tel que défini ci-dessus comprenant les étapes de
(a) ensemencement d'un milieu de culture approprié avec des cellules procaryotes exprimant la protéine ccdB et contenant un vecteur tel que défini ci-dessus,
(b) culture en fermenteur de la cellule ainsi transformée en l'absence d'antibiotique et
(c) récupération du vecteur produit au cours de l'étape (b).

Selon un autre aspect, la présente invention concerne un procédé de construction d'un vecteur auto réplicatif tel que défini ci-dessus comprenant les étapes de :
(a) construction d'un vecteur auto réplicatif comprenant un gène de résistance à un antibiotique fonctionnel encadré respectivement par une séquence 1 et une séquence 2, dans lequel les séquences 1 et 2 sont deux séquences chevauchantes de la séquence codant pour la protéine ccdA qui après recombinaison homologue reconstitue une séquence ccdA fonctionnelle,
(b) linéarisation dudit vecteur par utilisation d'une enzyme de restriction coupant le vecteur uniquement entre les séquences 1 et 2 et
(c) transformation d'une cellule procaryote exprimant la protéine ccdB,
(d) récupération des cellules procaryotes comprenant le vecteur auto réplicatif.

L'invention va être décrite en détails dans la description qui suit par référence aux figures annexées dans lesquelles :
La figure 1 est une représentation schématique du plasmide pSP1.
La figure 2 est une représentation schématique du procédé de construction d'un vecteur selon l'invention.

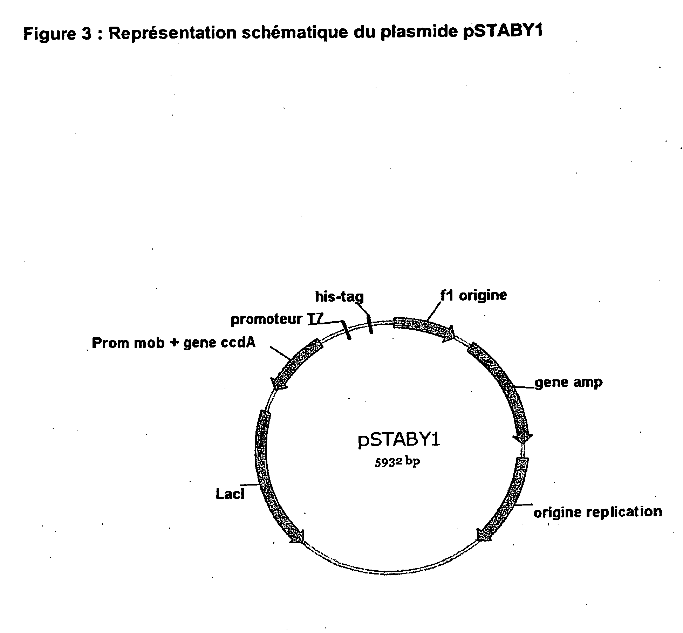

La figure 3 est une représentation schématique du plasmide pSTABY1.

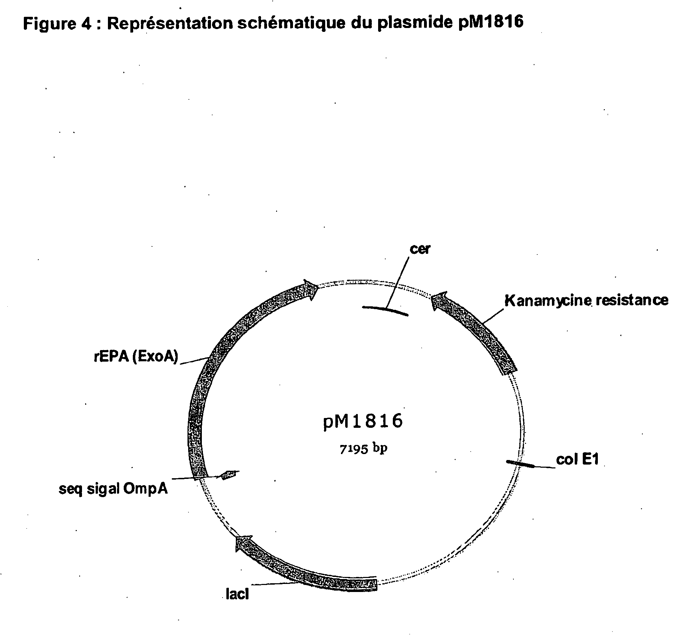

La figure 4 est une représentation schématique du plasmide PM1816.
La figure 5 est une représentation schématique du plasmide PM1800.
La figure 6 est une représentation schématique du plasmide pSP301.
La figure 7 est une représentation schématique du plasmide pSP2.
La figure 8 est une représentation schématique du plasmide pSP6.
La figure 9 est une représentation schématique du plasmide pSP4.
La figure 10 est une représentation schématique du plasmide pSP5.
La figure 11 est une représentation schématique du plasmide pSP3.
La figure 12 donne la cinétique de production de rEPA en fermenteur de 1 litre.
La figure 13 donne la cinétique de production de rEPA en fermenteur de 30 litres.

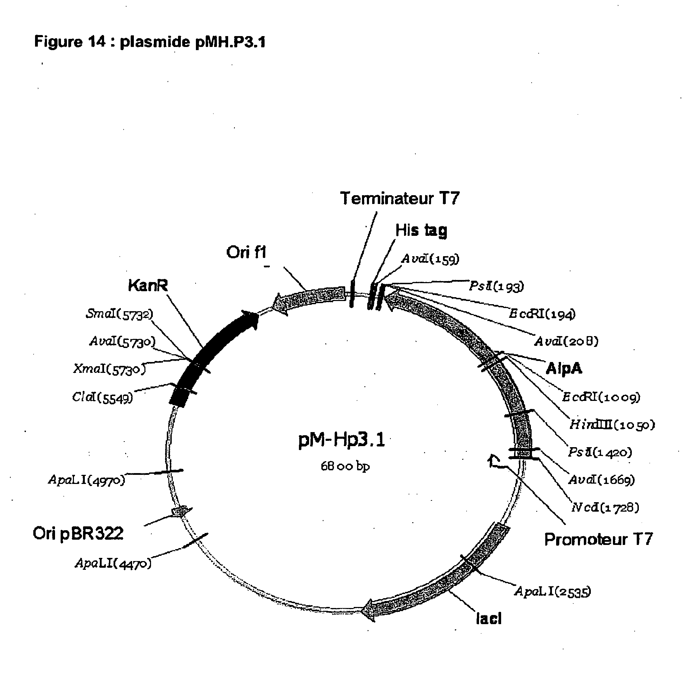

La figure 14 donne une représentation schématique du plasmide pMH.P3.1

Selon un premier aspect, la présente invention concerne donc un vecteur auto réplicatif comprenant :
- une séquence codant pour la protéine ccdA liée de façon fonctionnelle à un premier promoteur,
- la séquence du locus Cer et
- une séquence hétérologue, liée de façon fonctionnelle à un deuxième promoteur.

Par « vecteur auto réplicatif » on entend une molécule d'acide nucléique capable de se répliquer de façon autonome dans une cellule hôte. Le vecteur selon l'invention peut être un plasmide, un phagemide ou un bactériophage. Un vecteur auto réplicatif comprend donc une ou plusieurs séquences dirigeant ou contrôlant l'expression du produit des séquences d'acides nucléiques contenues dans ledit vecteur. Un tel vecteur comprend donc en particulier une origine de réplication fonctionnelle dans la cellule hôte transformée avec ledit vecteur. Le vecteur selon l'invention est avantageusement un plasmide en particulier un plasmide capable de se répliquer dans une cellule d'*E.coli.*

Toute origine de réplication classiquement utilisée dans les vecteurs auto réplicatifs peut être utilisée dans le cadre de la présente invention. L'origine de réplication confère une spécificité plus ou moins grande à l'égard de la cellule hôte et conditionne le nombre de copies dudit vecteur. L'origine utilisable pouvant être une origine à copie unique, à faible nombre de copies, ou à haut nombre de copies. Dans le cadre de l'utilisation du vecteur pour l'expression d'une protéine d'intérêt ou pour la production de vecteurs utilisables pour l'immunisation ADN ou la thérapie génique, l'origine de réplication est avantageusement une origine à haut nombre de copies (classiquement compris comme signifiant plusieurs centaines de copies) telle que colE1.

Les vecteurs auto réplicatifs selon l'invention sont des vecteurs dépourvus de gènes de résistance à un antibiotique.

On entend dans le cadre de la présente invention par « vecteur dépourvu de gène de résistance à un antibiotique » un vecteur qui ne contient aucun gène de résistance à un antibiotique ou qui comporte tout ou partie d'un gène de résistance à un antibiotique non fonctionnel. Avantageusement le vecteur selon l'invention ne comporte aucun gène de résistance à un antibiotique.

Le vecteur selon l'invention comprend comme unique marqueur de sélection une séquence codant pour une protéine ccdA. La protéine ccdA fonctionne en association avec une cellule hôte comprenant un gène fonctionnel codant pour la protéine ccdB.

Le gène codant pour le poison (ccdB) est introduit dans le chromosome bactérien de la cellule hôte: II code pour une protéine stable de100 acides aminés environ, qui se fixe sur la gyrase, induisant la mort de la bactérie.

Le gène codant pour l'antidote (ccdA) quant à lui, code pour une protéine instable d'environ 90 acides aminés, qui neutralise la protéine poison. Ce gène ccdA est introduit dans le vecteur selon l'invention sous contrôle d'un promoteur constitutif.

L'expression du gène poison est sous contrôle d'un promoteur qui peut être fortement réprimé par l'antidote ou le complexe poison-antidote. Par conséquent, lorsque le vecteur est présent dans la cellule hôte, le poison n'est pas produit. Par ailleurs, lorsque le vecteur est perdu, l'antidote est dégradé par une protéase et la production de poison induite provoque la mort de la cellule hôte. Ce système de sélection ccdA/ccdB est décrit en détails par Szpirer CY et Milinkovitch MC dans Biotechniques. 2005 May;38(5):775-81.

On peut également se référer au document WO99/58652 pour une description détaillée de ce système de sélection.

Ladite séquence codant pour la protéine ccdA est liée de façon fonctionnelle à un premier promoteur. Tout promoteur constitutif classiquement utilisé dans les vecteurs peut être utilisé dans le cadre de la présente invention. On utilisera avantageusement un promoteur non inductible. Il n'est pas nécessaire d'utiliser un promoteur fort. D'autre part le promoteur ne doit pas nécessairement être réprimé par ccdB ou le complexe ccdB/A. Avantageusement, on utilise le promoteur constitutif mob.

Le vecteur selon l'invention comprend en outre la séquence du locus Cer. Ladite séquence peut être insérée dans le vecteur selon une orientation quelconque et à une position quelconque. Ladite séquence Cer est décrite par Summers,D.K. et Sherratt,D.J. dans EMBO J. 7 (3), 851-858 (1988). Ladite séquence Cer est reproduite dans le séquence listing à SEQ ID N°1. Michaela E. Sharpe et al. dans Microbiology, 145, 2135-2144 (1999) et Claire Balding et al. dans Plasmid, 56(1), 68-73 (2006) décrivent les mécanismes de fonctionnement du locus Cer. D'autre part, Angelika Wegerer et al. a montré dans BMC Biotechnol. 2008 ; 8 : 2 que la combinaison des éléments Cer et ccdA/ccdB dans un même plasmide d'expression de la proteine eGF conduisait à déstabiliser le plasmide.

Le vecteur selon l'invention comprend de plus une séquence hétérologue liée de façon fonctionnelle à un deuxième promoteur.

Par séquence hétérologue on entend dans le cadre de la présente invention une séquence codant pour une protéine thérapeutique ou utilisable à des fins de diagnostic ou pour un antigène vaccinal, ainsi que pour toute protéine d'intérêt commercial ou industriel ou une séquence d'un gène hétérologue ayant un intérêt dans la vaccination ADN ou la thérapie génique.

A titre d'exemples de protéines thérapeutiques, on peut citer en particulier : les dérivés sanguins, les hormones, en particulier l'hormone de croissance, les lymphokines, les protéines codant pour une activité enzymatique capable d'opérer la transformation d'une pro-drogue en drogue en particulier dans le cadre d'un protocole de traitement du cancer tel que décrit par exemple dans le tableau 3 de la revue générale de Kratz et al ChemMedChem. 2008 Jan;3(1):20-53.

A titre d'exemples d'antigène vaccinal on peut citer les protéines utilisables dans un programme d'immunisation ainsi que les gènes utilisables dans le cadre d'une vaccination ADN, en particulier les protéines de surface d'un pathogène bactérien mais également les protéines d'enveloppe virale, les protéines de parasites ou encore les marqueurs de surface de cellules tumorales.

A titre d'exemple de séquences utilisables dans le cadre d'une thérapie génique on peut citer, les séquences utilisables dans le cadre du traitement des maladies génétiques telles que la mucoviscidose.

Une séquence hétérologue codant pour une enzyme par exemple une enzyme d'intérêt industriel telle que la benzonase, la trypsine ou encore toute molécule capable d'intervenir dans un procédé de biocatalyse peut être insérée dans un vecteur auto réplicatif selon l'invention.

Plus spécifiquement, on peut citer à titre d'exemple non limitatif d'antigène vaccinal pouvant être produit avec le vecteur selon l'invention : l'exoprotéine A de *Pseudomonas aeruginosa* détoxifiée par exemple par délétion du résidu Glu 553 (rEPA), l'antigène tbpB (transferin binding protein) de N. meningitidis B ( Legrain et al Protein Expr Purif. 1995 Oct;6(5):570-8.), la protéine AlpA d'hélicobacter pilory, l'hémaglutinine du virus Influenza HA, telle que par exemple la séquence SEQ ID NO 5 ou les séquences correspondantes issues d'autres souches du virus de la grippe , la séquence codant pour CFTR, telle que par exemple SEQ ID NO 6, avantageusement sans introns,(Babenko,A.P.J. Biol. Chem. 283 (14), 8778-8782 (2008)), les antigènes provenant de la forme « sporozoïte » de *Plasmodium falciparum,* (tels que la protéine majeure de surface du sporozoïte (circumsporozoite protein), LSA3, ou l'antigène Pfs 16, ainsi que des antigènes provenant de la forme « mérozoïte » de *Plasmodium falciparum* (tels que l'antigène MSP1, MSP2, MSP3, EBA-175, Rhop-1, Rhop-2, Rhop-3, RAP-1, RAP-2, RAP-3, Pf155/RESA ou AMA-1). Les séquences codant pour ces protéines sont connues et diponibles sur différentes bases de données. Par exemple, la séquence entière du gène LSA 3 est longue de 12240 paires de bases et code pour une protéine de 1558 acides aminés. Les séquences nucléotidique et protéique sont décrites dans la banque de données EMBL sous les numéros d'accession AE001424 et uniprot 096275-PLAF7. Comme séquence hétérologue, on peut utiliser la séquence codant pour la protéine entière pour des fragments de cette protéine, comme ceux décrits dans WO 02/38176. Habituellement, on utilise la protéine entière (qui peut contenir une ou plusieurs mutations ponctuelles pour tenir compte des variations qui existent entre les souches de Plasmodium falciparum) ou un fragment de cette protéine dont la séquence d'acides aminés a une identité d'au moins 80%, en particulier au moins 90 %, notamment au moins de 95 à 99% par rapport à la séquence entière décrite dans uniprot 096275-PLAF7.

Le système selon l'invention est particulièrement adapté à la production de protéines pour lesquelles la glycosylation est absente ou non indispensable à leur efficacité ou à l'induction d'une réponse immunitaire, par contre les séquences codant pour les protéines glycosylées peuvent être utilisées par exemple en vaccination ADN.

Tout promoteur constitutif ou inductible classiquement utilisé dans les vecteurs peut être utilisé dans le cadre de la présente invention comme deuxième promoteur. On peut citer à titre d'exemple les promoteurs T7, T5, arabinose, lac, Trp ou tout autre promoteur issu de microorganismes capable de fonctionner dans une cellule hôte procaryote, en particulier *Escherichia coli.* Dans le cas de vecteurs utilisés pour la thérapie génique ou l'immunisation ADN, l'expression du gène d'intérêt peut être sous contrôle d'un promoteur fonctionnant dans des cellules eucaryotes comme le promoteur du CMV ou celui de SV40 ou encore des promoteurs possédant une spécificité cellulaire.

Le vecteur selon l'invention peut contenir également des séquences régulant l'expression telles que par exemple une séquence de type terminateur régulant la transcription, une séquence signal permettant l'exportation de la protéine exprimée vers le compartiment périplasmique de la cellule hôte ou la sécrétion de ladite protéine dans le surnageant de culture des cellules hôtes ainsi qu'un site de clonage multiple. Ces séquences sont bien connues de l'homme de l'art.

Le vecteur selon l'invention peut être construit par toute technique classique de génie génétique.

Bien que l'utilisation de la sélection ccdA / ccdB soit possible dès les premières étapes de construction du vecteur il est possible de maintenir une pression de sélection par antibiotique jusqu'à la phase finale du procédé de construction du vecteur. L'élimination du gène de résistance à un antibiotique peut ensuite être réalisée par simple digestion par une enzyme de restriction au niveau de sites uniques, encadrant le gène de résistance à l'antibiotique, puis re-ligation du vecteur sur lui-même.

Le couple final cellule hôte/vecteur est obtenu après transformation de la cellule procaryote avec le plasmide re-ligué sur lui-même. Un moyen de vérifier l'élimination dudit gène consiste alors à repiquer les colonies obtenues, après transformation, en parallèle, sur des boites contenant ou non l'antibiotique ou encore à vérifier l'absence dudit gène par PCR, cartographie de restriction ou toute autre méthode appropriée. Dans tous les cas cette vérification supplémentaire s'avère fastidieuse.

Les inventeurs ont mis en évidence un autre système de construction du vecteur selon l'invention ayant l'avantage de conduire à une sélection aisée des clones recherchés.

Selon un deuxième aspect, la présente invention a donc pour objet un procédé de production d'un vecteur auto réplicatif tel que défini ci-dessus comprenant les étapes de :
(a) construction d'un vecteur auto réplicatif comprenant un gène de résistance à un antibiotique encadré respectivement par une séquence 1 et une séquence 2, dans lequel les séquences 1 et 2 sont deux séquences chevauchantes de la séquence ccdA qui après recombinaison homologue reconstitue une séquence ccdA fonctionnelle,
(b) linéarisation dudit vecteur par utilisation d'une enzyme de restriction présentant un site de restriction uniquement entre les séquences 1 et 2,
(c) transformation d'une cellule procaryote exprimant une protéine ccdB fonctionnelle et
(d) récupération des cellules contenant le vecteur selon l'invention.

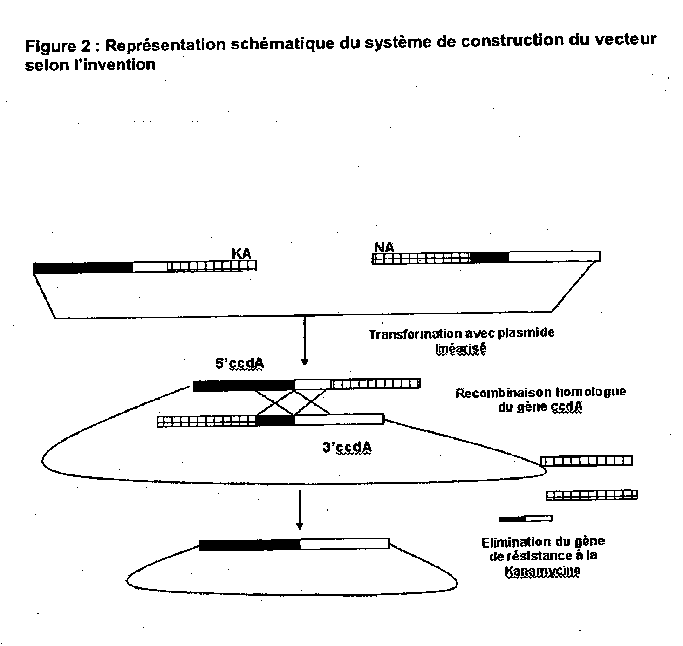

Le nouveau procédé selon l'invention utilise un évènement de recombinaison qui permet en une seule et unique étape d'éliminer le gène de résistance à l'antibiotique et d'assembler un gène ccdA sous sa forme fonctionnelle. En résumé, le gène ccdA est cloné sous forme de 2 éléments séparés et individuellement non-fonctionnels situés de part et d'autre du gène de résistance à un antibiotique. Les parties dites 5' et 3' de ccdA sont définies de façon à contenir une séquence commune d'une longueur par exemple de 200 nucléotides. Tant que le gène de résistance à l'antibiotique est présent l'ensemble ccdA est non-fonctionnel. Après digestion par une enzyme de restriction possédant un site situé uniquement entre les séquences 1 et 2, par exemple à l'intérieur du gène de résistance à l'antibiotique, un fragment d'ADN linéaire est obtenu. Par transfection de cellules procaryotes, par exemples de cellules d'E. *coli* compétentes, cette molécule d'ADN peut se re-circulariser par recombinaison homologue des fragments chevauchants de ccdA. Cette étape permet d'une part d'éliminer le gène de résistance à l'antibiotique et d'autre part une sélection des clones contenant le vecteur selon l'invention dans la mesure où le gène ccdA ne peut être fonctionnel qu'après élimination du gène de résistance à l'antibiotique. Une représentation schématique du procédé selon l'invention est donnée à la figure 2.

Pour la mise en oeuvre du procédé ci-dessus on peut se référer à un ouvrage de référence en matière de génie génétique tel que Maniatis et al « Condensed Protocols from Molecular Cloning: A Laboratory Manual" de Joseph Sambrook et David W. Russell
qui décrit l'ensemble des conditions opérations à utiliser pour les étapes de construction d'un vecteur, linéarisation, transformation et récupération des clones. Le vecteur auto-réplicatif peut ensuite être facilement récupéré à partir des cellules transformées par tout procédé classique bien connu de l'homme de l'art.

S'agissant en particulier de la recombinaison homologue, la taille de la séquence chevauchante peut varier dans une large mesure. Des séquences se chevauchant sur seulement 5 nucléotides peuvent être utilisées. Le seul élément important dans la sélection des séquences 1 et 2 est que les deux séquences après recombinaison homologue reconstituent une séquence ccdA fonctionnelle, par exemple la séquence ccdA complète. Elles doivent d'autre part encadrer le gène de résistance à l'antibiotique pour permettre l'élimination de ce dernier.

Tout vecteur auto réplicatif tel que défini ci-dessus peut être avantageusement produit par le nouveau procédé selon l'invention.

Selon un autre aspect, la présente invention concerne donc une cellule procaryote contenant un vecteur auto réplicatif tel que défini ci-dessus.

Toute cellule procaryote exprimant une protéine ccdB fonctionnelle peut être utilisée dans le cadre de la présente invention. Une protéine ccdB est fonctionnelle si elle st capable d'inhiber de façon significative l'action de la gyrase c'est-à-dire si elle est capable d'induire un effet létal sur la cellule procaryote. On peut donc utiliser la protéine ccdB entière ou des fragments de cette dernière qui sont capables d'entrainer la mort cellulaire en l'absence de protéine ccdA. On utilisera avantageusement une cellule d'E.coli exprimant la protéine ccdB, en particulier une cellule d'E.Coli dans laquelle la séquence codant pour la protéine ccdB a été insérée dans le génome bactérien. L'insertion de la séquence ccdb dans le génome bactérien peut être réalisée par tout procédé bien connu de l'homme de l'art pour ce faire.

Selon un autre aspect la présente invention concerne donc un procédé de production d'une protéine hétérologue comprenant les étapes de :
(a) ensemencement d'un milieu de culture approprié avec des cellules procaryotes exprimant la protéine ccdB telles que définies ci-dessus et contenant un vecteur tel que défini ci-dessus,
(b) culture en fermenteur en l'absence d'antibiotique et
(c) récupération de la protéine hétérologue produite au cours de l'étape (b) à partir du surnageant ou du culot cellulaire.

La présente invention a également pour objet un procédé de production d'un vecteur auto réplicatif tel que défini ci-dessus comprenant les étapes de :
(a) ensemencement d'un milieu de culture approprié avec des cellules procaryotes exprimant la protéine ccdB telles que définies ci-dessus et contenant un vecteur tel que défini ci-dessus,
(b) culture en fermenteur de la cellule ainsi transformée en l'absence d'antibiotique et
(c) récupération du vecteur produit au cours de l'étape (b).

Classiquement, pour mettre en oeuvre ces procédés, les cellules procaryotes, avantageusement des cellules d'E. coli, proviennent d'un lyophilisat ou d'un congelât ; elles sont ensemencées dans un volume de milieu de culture n'excédant généralement pas 1 litre. Au bout d'une nuit de culture ou lorsque la densité optique du milieu est suffisante, on transfert cette première culture dans un second milieu de culture identique ou différent du premier, mais dont le volume peut être au moins 10, en particulier au moins 20 et notamment au moins 30 fois ou 60 fois plus grand. Cette deuxième culture, inoculée entre 1 à 10% est réalisée dans un fermenteur de 1 à 10 000 litres, en particulier dans un fermenteur de 30 à 10 000 litres, notamment dans un fermenteur de 30 à 500 litres, tel que par exemple 50 à 100 litres.

Dans le cadre de la production de produits d'intérêt commercial, tels que des enzymes, des fermenteurs de plus grands volumes peuvent être utilisés. Des fermenteurs de 1000 à 100 000 litres, par exemple 60 000 litres peuvent être utilisés pour cette dernière étape de culture.

On utilise habituellement pendant la durée de la culture, par exemple de 6h à 24h, une température de l'ordre de 25°C à 42°C, en général de 30 à 37°C, un pH de 6.5 et 7.5, une agitation comprise entre 200 et 1500 rpm, une pression de 0 à 500 mb, une pO2 régulée entre 20 et 50% et un débit d'air équivalent de 1 à 2 volumes d'air par volume de milieu et par minute qui peut être enrichi en oxygène représentant 0 à 50% du débit total de gaz.

Tout milieu de culture approprié pour la croissance de la cellule procaryote utilisée peut être utilisé. Un grand nombre de milieu de culture sont décrits dans la littérature et disponibles dans le commerce. On peut citer à titre d'exemple les milieux de culture des cellules d'E. coli utilisés dans le cadre de la présente invention.

A la fin de la phase exponentielle de croissance cellulaire, il est possible d'amplifier encore la biomasse en la transférant dans un autre fermenteur de plus grande capacité en utilisant la même procédure. Les volumes de culture peuvent atteindre voire dépasser les 10000 litres. L'étape de culture en fermenteur est généralement réalisée selon le mode « Batch ». Il est également possible d'adopter d'autres modes de culture en fermenteur tel que le mode « fed batch ». Dans un tel cas de figure, on rajoute au milieu un complément nutritif comprenant des carbohydrates pendant la phase exponentielle de croissance de façon à prolonger la multiplication bactérienne et à obtenir en fin de phase exponentielle une densité cellulaire plus importante. La quantité de carbohydrate ajoutée est évaluée en fonction de la densité cellulaire et du taux de croissance de la cellule. Le mode de culture en fed batch est particulièrement avantageux dans le cadre de la présente invention.

Dans le cas de la production d'une protéine d'intérêt, on prélève en fin de culture le sumageant si la protéine d'intérêt est sécrétée dans ce dernier puis on la purifie. Si la protéine n'est pas sécrétée dans le sumageant, on récupère le culot cellulaire et on extrait puis purifie la protéine d'intérêt à partir de ce dernier. Tout procédé décrit dans la littérature comme étant approprié pour ce faire peut être utilisé dans le cadre de la présente invention. On peut se référer par exemple à l'ouvrage « protein purification » 2nd édition, Janson, J-C & Ryden, L, 1998.

Selon un mode de réalisation particulier, la souche procaryote utilisée est une souche d'E. *coli* exprimant la protéine ccdB.

Le procédé est avantageusement utilise pour la production des protéines identifiées ci-dessus en particulier pour la production de rEPA.

Dans le cas de la production d'un vecteur selon l'invention, le vecteur est récupéré à partir du culot cellulaire et purifié. Tout procédé connu, classiquement utilisé dans la littérature pour ce faire peut être utilisé dans le cadre de la présente invention.

Les inventeurs ont mis en évidence que le nouveau vecteur selon l'invention conduit à une augmentation significative de la quantité de protéines d'intérêt ou de vecteur d'intérêt produite. Le vecteur selon l'invention conduit à un confinement amélioré dans la cellule procaryote exprimant la protéine ccdB.

Le confinement du vecteur dans une cellule procaryote peut être facilement évalué par exemple par détermination, à un temps t de la culture, de la proportion de cellules ne contenant plus de vecteur. Classiquement cette détermination se fait par évaluation par PCR de la présence d'ADN du vecteur et comptage.

Par amélioration du confinement dans la cellule procaryote on entend une diminution d'au moins 20% de cellules procaryote ayant perdu le vecteur, par comparaison avec un système vecteur/cellule procaryote dans lequel le système ccdA/Cer est remplacé par un système classique de sélection par un gène de résistance à un antibiotique et ce dans les mêmes conditions de culture après une durée de culture d'au moins 18 heures et par utilisation de la même méthode de détermination. La diminution mesurée est avantageusement d'au moins 30%, en particulier d'au moins 50%, notamment d'au moins 70%.

Le vecteur selon l'invention conduit à une production améliorée après culture en fermenteur de protéines d'intérêt ou vecteurs d'intérêt.

Par amélioration de la production en fermenteur on entend une augmentation d'au moins 10% de la quantité de protéine d'intérêt produite par rapport à la quantité de protéines totales, telle que déterminée, après 18H de culture en fermenteur, par analyse densitométrique d'un gel d'électrophorèse coloré, soit au bleu de comassie, soit au nitrate d'argent, par comparaison avec une production mise en oeuvre dans les mêmes conditions mais utilisant un gène de résistance à un antibiotique comme seul système de sélection (sans fragment cer). Un tel test est décrit dans les exemples qui suivent.

Lorsque l'on s'intéresse à la production du vecteur en tant que tel, par exemple à des fins de vaccination ADN, par amélioration de la production en fermenteur, on entend une augmentation d'au moins 10% en poids de la quantité d'ADN du vecteur telle que déterminée, après 18H de culture en fermenteur de la souche hôte contenant le vecteur d'intérêt, par dosage ADN par mesure à 260nm sur un échantillon de vecteur purifié par comparaison avec une production mise en oeuvre dans les mêmes conditions et dosée dans les mêmes conditions mais utilisant un gène de résistance à un antibiotique comme seul système de sélection (sans fragment cer).

### Exemple 1 - Construction d'un vecteur selon l'invention exprimant la protéine rEPA

Dans un premier temps, le promoteur mob et le gène ccdA (SEQ ID NO 7) ont été amplifiés par PCR avec les amorces NotccdA+(SEQ ID N02) et EcoRIccdA-(SEQ ID N03) à partir du plasmide pStaby1 (5932 pb commercialisé par la société Delphi genetics) utilisé comme matrice dans les conditions suivantes:
Plasmide pStaby1 100ng, Tampon « High Fidelity »X10 (Invitrogen)10µl ; mélange dNTP 125µM; amorce 5' 1µM; amorce 3' 1µM; MgSO4 50mM 3µl; Taq polymérase « High Fidelity »(Invitrogen) 2,5 U ; H2O qsp 100µl en mettant en oeuvre le programme PCR ci-dessous.

### Programme PCR

| **Etape** | **Température** | **Temps** | **Nombre de cycles** |
|---|---|---|---|
| Dénaturation | 97°C | 30sec | 30 |
| Hybridation | 56°C | 1min | |
| Polymérisation | 72°C | 30sec | |
| Polymérisation | 72°C | 7min | 1 |

Les fragments d'ADN sont purifiés par électrophorèse préparative en gel d'agarose 1% dans du tampon Tris-Acétate-EDTA 1X. Les bandes correspondant aux fragments d'intérêt sont découpées et l'ADN récupéré par électro élution est purifié avec le kit Qiaquik de la société Qiagen.

Le fragment PCR ccdA est ensuite digéré par 45 U de Notl et 30U de EcoRI pour 2250 ng d'ADN. En parallèle 5 µg de plasmide PM1816 (7195 bp) est digéré avec 30 U de Notl et 20 U d'enzyme EcoRI. Les enzymes de restriction de Invitrogen ou de New England Biolabs sont utilisées avec les tampons correspondants X10 commercialisés par le fournisseur. Les réactions ont lieu 2 h à 37°C.

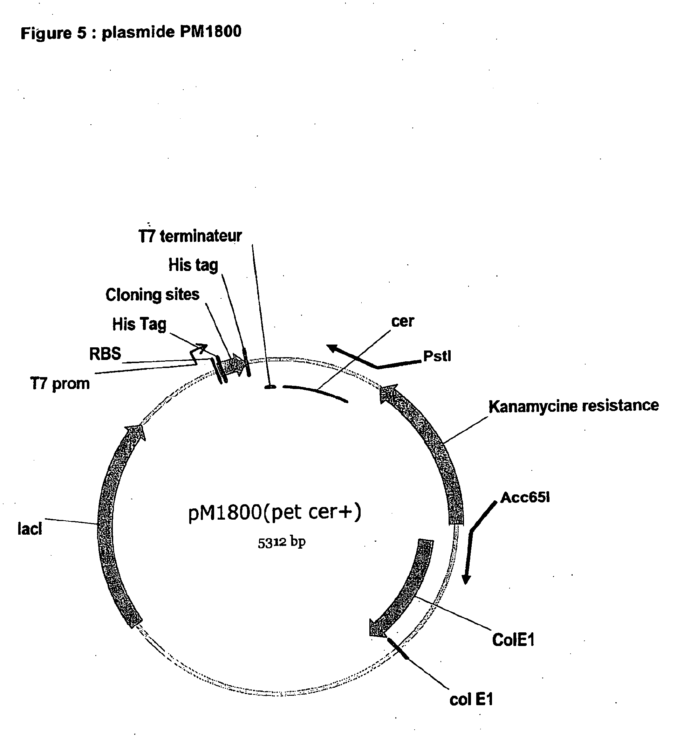

Le plasmide PM1816 a été construit à partir du plasmide pET28c (Novagen) qui a été amplifié par PCR pour supprimer l'origine F1. Cette amplification a permis de créer les sites Pacl et Ascl puis le fragment Cer a été cloné entre ces mêmes sites de restriction. Le vecteur ainsi créé a été nommé pM1800 (cf. figure 5 pour une représentation schématique). Enfin, la séquence SEQ ID N09 (contenant RBS-Omp A-rEPA) a été clonée entre les sites Xbal et EcoRI, créant ainsi le vecteur pM1816.

La ligation des fragments PCR et du vecteur digéré, a été réalisée avec le Kit de ligation rapide d'ADN de Roche en 5min à température ambiante, avec un excès molaire de fragments de 6X. Le nouveau vecteur obtenu est nommé pM1816+ccdA.

Afin d'éliminer le gène Kanamycine, le plasmide pM1816+ccdA a été digéré par les enzymes ClaI et Ascl. Les extrémités cohésives ainsi libérées ont été traitées avec l'exonucléase « Mung Bean ». (New England BioLabs.) utilisée à 10U/µl, à 30°C pendant 1h. La re-ligation du vecteur aux extrémités franches a été réalisée avec l'enzyme : T4 DNA Ligase New England BioLabs. (400U/µL) pendant une nuit à 16°C délétant ainsi 690 pb sur 816 pb du gène Kanamycine. Les bactéries CYS21 (souche commercialisée par Delphigenetics, ayant pour génotype : F- Cmr mcrA endA1 Δ(mrr-hsdRMS-mcrBC° (restriction-modification), Φ80lacZ, ΔM15, ΔlacX74, recA1,deoR, Δ(ara,leu)7697, galU, galK, nupG, rpsl, ccdB+ (contenant le gène ccdB dans leur génome)) sont électrotransformées avec le produit de ligation, puis étalées sur boite gélosée sans antibiotique.

Les clones ainsi obtenus ont été testés en minipréparations d'ADN et un clone ayant le bon profil de digestion a été préparé en maxipréparation d'ADN puis séquencé.

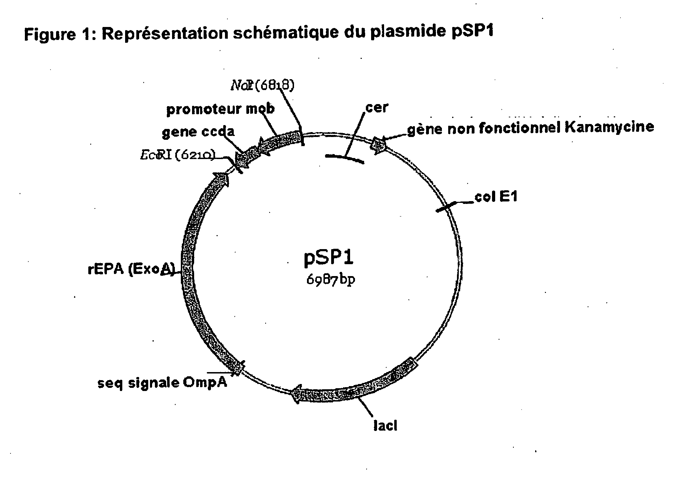

Le nouveau vecteur obtenu est nommé pSP1. Une représentation schématique de ce plasmide est donnée à la figure 1.

### Exemple 2 Comparaison de l'expression de la protéine rEPA obtenue avec les vecteurs pSP1, PM1816 et PM1816+ccdA à l'échelle du laboratoire.

Les systèmes vecteurs/ cellules hôtes testés sont décrits dans le tableau ci-dessous.

| Vecteur | Cellule hôte | Milieu gélosé utilisé |
|---|---|---|
| PM1816 | BL21λDE3 | LB+Kanamycine 50µg/ml |
| PM1816+ccdA | BL21λDE3 | LB+Kanamycine 50µg/ml |
| pSP1 | SE1 | LB |

Les bactéries SE1 sont dérivées des cellules BL21λDE3 et possèdent le gène poison ccdB dans leur génome. Elles sont commercialisées par la société Eurogentec et sont caractérisées par le génotype suivant : F-, CmR, ompT, hsdSB (restriction-, modification), gal, dcm, DE3(lacl, T7 polymérase sous le control du promoteur PlacUV5) Ion, ccdB+.

Les cellules BL21λDE3, dérivées des cellules *E coli* B, sont commercialisées par Invitrogen et sont caractérisées par le génotype suivant : F⁻ ompT hsdS_{B} (r_{B}⁻ m_{B}⁻) gal dcm(DE3).

Le milieu LB est disponible dans le commerce chez Invitrogen. Il est constitué par litre de milieu de 10g de tryptone, 5g d'extrait de levure, 10g de NaCl et qsp 1 L avec H20 tamponnée à pH 7,5 par ajout de NaOH 5N.

Les cellules procaryotes ont été transformées par électroporation selon les conditions ci dessous :
Les cellules d'E.coli BL21λ DE3, sont incubées 30min dans la glace après ajout de 8µL de produit issu de la ligation. La transformation est mise en oeuvre par choc thermique de 45 secondes à 42°C.

Les bactéries transformées sont reprises dans du milieu SOC (constitué par litre de milieu de 20 g de tryptone, 5g d'extrait de levure, 0,5g de NaCl, 10mL d'une solution à 250mM de KCI, 5mL d'une solution à 2M de MgCl₂, 20mL d'une solution à 1 M de glucose et qsp 1 L d'H₂O à pH7 avec NaOH 5N) puis incubées 1 h à 37°C et étalées sur milieu gélosé LB+ kanamycine 50µg/ml, avant incubation sur la nuit à 37°C

Les bactéries SE1 sont incubées 10 min dans la glace après ajout de 2µL du produit issu de la ligation. La transformation par électroporation a lieu grâce à l'appareil Gene pulser™ (Biorad) dans une cuve comprenant 2 électrodes distantes de 1 mm, sous un courant de1,7kV. Les bactéries transformées sont reprises dans du milieu SOC puis étalées sur milieu gélosé LB sans antibiotique, avant incubation sur la nuit à 37°C

Un essai d'expression a été réalisé dans les conditions suivantes :
La pré culture est réalisée par ensemencement de 5 ml de milieu LB avec une colonie piquée sur une boite de pétri sur laquelle ont été étalées les bactéries transformées et incubation pendant la durée d'une nuit à 37°C sous agitation. Le lendemain, 50 ml de milieu LB ou LB+ kanamycine sont ensemencés avec 500µL de ladite pré culture. Cette culture est mise sous agitation à 37°C, jusqu'à ce que la DO à 600 nm soit comprise entre 0,4 et 0,8. A ce stade la culture est partagée en 2 fois 25ml dans 2 erlens. Dans un erlen est ajoutée une solution de IPTG à 0,01 M final. L'autre Erlen servira de témoin non induit.

Les deux cultures sont remises sous agitation pendant 4h à 37°C. La DO est ensuite mesurée, et un échantillon de 1 ml est prélevé dans les 2 erlens.

Les échantillons issus des tests d'expression dans *E Coli* ont été repris en bleu dénaturant 2X (100mM Tris-HCl pH6,8 ; 20% Glycérol ; 4% SDS ; 0,2% Bleu de bromophénol ; 200mM β-mercaptoéthanol) afin de concentrer jusqu'à une DO de 10 par ml. 20µL sont ensuite déposés sur gel Tris-Glycine 4-20% (PAGEr^{®} Duramide^{®} Precast Gels, Cambrex).On a utilisé comme marqueur de poids moléculaire le marqueur d'Invitrogen 10748-010.

Après les dépôts, le gel est mis à migrer 2 heures dans du tampon de migration (250mM Tris-HCl ; 1,92M Glycine ; 1% SDS) sous une tension de 175V. Le gel est alors, soit révélé au bleu de Coomassie (rinçage 30min dans l'eau distillée, coloration 30 min dans le bleu de Coomassie, décoloration dans un mélange acide acétique (10%), méthanol (30%), eau distillée (60%)), soit transféré sur membrane de nitrocellulose, par électrotransfert semi-sec (2117 Multiphorll Electrophoresis Unit, LKB Bromma) 2 heures à 65mA.

Après électrotransfert, la membrane de Western Blot est incubée 1 heure dans une solution de PBS-lait à 3%. La membrane est ensuite lavée trois fois dans du PBS-Tween 0,05% avant d'être incubée 1 heure à température ambiante avec le premier anticorps spécifique correspondant à un sérum de lapin anti-rEPA. La membrane est ensuite lavée trois fois dans du PBS-Tween 0,05% puis incubée 1 heure à température ambiante avec une IgG de chèvre anti-IgG de lapin conjuguée à HRP (Zimed ref 65-6120) dilué au 1/1000 en PBS. Après 3 lavages en PBS-Tween 0,05% la membrane est incubée 15min avec le substrat peroxydase (« Peroxydase Opti-4CN Substrate « Kit, Biorad) et rincée à l'eau distillée.

Les résultats obtenus peuvent être résumés de la façon suivante :
La quantité de rEPA produite avec le système pSP1 dans les bactéries SE1 est évaluée à 12% (des protéines totales) et celle produite avec le système classique PM1816 en sélection kanamycine 14% des protéines totales, soit des taux tout à fait équivalents. On remarque une bande fortement révèle à environ 67kd correspondant à la protéine rEPA, et ceci de manière plus intense dans les échantillons « induits »

Il n'y a pas différence visible dans ces conditions entre le système classique PM1816 et le système pSP1.

Pour analyser la stabilité du plasmide pSP1 dans les bactéries SE1, après une culture de 4h post induction les bactéries sont étalées sur milieu LB gélosé. 88 clones ainsi obtenus ont été ensuite analysés en minipreparation d'ADN

Tous les clones ayant poussé en milieu liquide, possèdent le plasmide.

On peut donc conclure à la fonctionnalité du gène ccdA. Les bactéries SE1 ne pouvant pas survivre sans le gène antidote ccdA contenu dans le plasmide et à la très bonne stabilité du plasmide lors de l'essai d'expression.

### Exemple 3 Expression de la protéine rEPA en fermenteurs de 1 litre et 30 litres

L'objectif de ce travail est d'évaluer le vecteur selon l'invention pour la production de protéines recombinantes à l'échelle pilote.

### Fabrication d'un lot de semence expérimental (stock glycérol).

Après transformation de la souche SE1 (Eurogentec), par le plasmide pSp1 comme cela est décrit dans l'exemple précédent, le niveau d'expression est évalué à petite échelle.

50ml de milieu LB sont inoculés au 1:100 avec les cellules SE1 contenant le plasmide pSp1 et incubés pendant la durée d'une nuit à 37°C.

Quand la DO (600nm) atteint la valeur de 0.8, l'IPTG (1mM) est rajouté pour l'induction.

Après 4h d'incubation, 1ml de suspension bactérienne est prélevé, centrifugé et le culot est resuspendu dans 75µl de tampon Tris 50 mM pH 7.4, EDTA 1mM et saccharose 20%. Puis le volume est ajusté à 750 µl avec du tampon Tris-HCl 50mM pH 7.4 EDTA 1 mM. L'échantillon est déposé sur gel de polyacrylamide. Après coloration par le bleu de Coomassie, les bandes de protéines sont scannées avec un densitomètre GS-710 de Biorad. Dans ces conditions on observe que la quantité de protéine d'intérêt représente 12% des protéines totales.

Après évaluation de l'expression à petite échelle, un lot de semence expérimental comprenant 45 ampoules a été préparé selon le mode opératoire suivant : le clone évalué est étalé en masse sur gélose Trypcase soja et incubé à 37°C pendant 24h. La nappe bactérienne est ensuite reprise par 2 ml du milieu LB2X pour inoculer 100 ml de milieu LB2X modifié dans un Erlen de 500 ml afin d'obtenir une DO600nm initiale comprise entre 0,1 et 0,2 unités. L'erlen est agité à 175 rpm à 37°C jusqu'à l'obtention d'une DO600 comprise entre 3 et 5. La suspension est refroidie puis centrifugée à 4000 rpm pendant 30 min à 4°C. Le surnageant est éliminé et le culot est repris avec 50ml de milieu de congélation refroidi au préalable. La suspension est ensuite répartie dans des Cryotubes Nunc à raison de 1ml par tube puis stockée à une température ≤-70°C à en présence de glycérol.

Le milieu de préculture LB2X modifié contient pour 1L de mélange: 30 g extrait de levure,: 2 ml MgCl₂ 1M, 10 g NaCl :, 10 ml Tris 2M pH 7,5 ± 0,1 :, qsp avec eau UF. Stérilisation à la chaleur (45 min / 121 °C : cycle liquide).

Le milieu de congélation est composé à 50% de milieu LB2X modifié et 50% d'une solution de congélation. La solution de congélation contient pour 1 litre de mélange 10ml de MgSO4 1 M, 200g de glycérol 100% et 5g de NaCl QSP avec eau UF. Stérilisation par filtration 0.22µm.

### Evaluation en fermenteur

500ml du milieu de fermentation GluSKYE (contient pour 1L : 40 g d'extrait de levure, 3 ml MgCl₂ 1 M, 0,53 g K₂SO₄: 1 g NaCl, 0,84 g K₂HPO₄, 44 g glucose, Qsp avec eau UF (30 min / 121°C)) sont inoculés avec 25ml d'une pré culture agitée à 175 rpm pendant 16h et maintenue à une température de 37°C. Cette pré culture contenant le milieu LB 2X a été au préalable ensemencée avec 500µl d'une ampoule de lots de semence, stockée à -70°C et contenant la bactérie productrice sous forme de congelât.

L'équipement de fermentation utilisé est le Biostat Q (Sartorius) qui comporte 4 cuves de 1 L (500 ml utiles) reliées à une armoire de régulation et à un système de supervision qui enregistre et archive les données. Les paramètres de fermentation sont les suivants : pH 7.0 ; température 37°C, agitation initiale de 200 rpm, aération initiale de 0.5L d'air /min, pO2 maintenue à 30%. L'induction de la protéine r-EPA s'effectue par addition d'IPTG 1mM lorsque la DO à 600nm est comprise entre 25 et 35 unités. La culture est ensuite arrêtée après 3h d'induction.

Une variante du procédé consiste à induire la synthèse de rEPA à un stade plus précoce de la culture (entre 1 et 3 unités D.O au lieu de 25-35.), afin de tester la stabilité du plasmide dans des conditions plus sélectives. Dans ce même contexte, une autre variante consiste à prolonger la durée d'induction jusqu'à 20h.

Une première évaluation a été réalisée en fermenteurs Biostat Q (BBI) configuré en 4 cuves de 0.5 Litre utile, paramétrables individuellement vis à vis du pH, de la pO2 et de la température. La composition du milieu ainsi que la valeur des paramètres physiques sont identiques à ceux retenus pour le procédé basé sur l'utilisation de la kanamycine. D'autre part,4 cultures sont réalisées, la souche témoin *E. coli* BL21/pM1816 (Km système) et la souche du nouveau système *E. coli* SE1/pSP1, sont testées dans 2 conditions d'induction (induction rapide et induction tardive).

L'évaluation à plus grande échelle a été effectuée sur un fermenteur de 30L Applikon en respectant les mêmes principes.

Des échantillons ont été prélevés régulièrement pour documenter les paramètres suivants: OD (600nm), poids sec cellulaire CDW (g/l), numération (CFU/ml), confinement du plasmide (%), quantité de rEPA (PAGE, ELISA)

Chaque échantillon de culture est traité par un choc osmotique pour libérer le produit présent dans le périplasme. Puis chaque extrait est analysé sur PAGE.

### Suivi de la stabilité du plasmide:

Le suivi est assuré par l'analyse individuelle du contenu plasmidique de chaque colonie. A partir d'échantillons de culture dilués et déposés sur boîte, après incubation une centaine de colonies sont reprises individuellement dans des microplaques (96 trous) contenant du milieu LB. La plaque est ensuite placée dans un système d'extraction plasmidique robotisée (Qiagen bioroBot ^{R} Systems) pour une incubation de 24h pour permettre une amplification des bactéries. Elles sont ensuite centrifugées, lysées et filtrées puis les échantillons sont déposés sur gel d'agarose pour l'électrophorèse. Après migration et coloration, on vérifie la présence de plasmide sur le gel.

Dans le cas du système PM1816, 100 colonies individuelles sont repiquées sur milieu LB agar supplémenté en kanamycine à 50 µg/ml concentration finale.

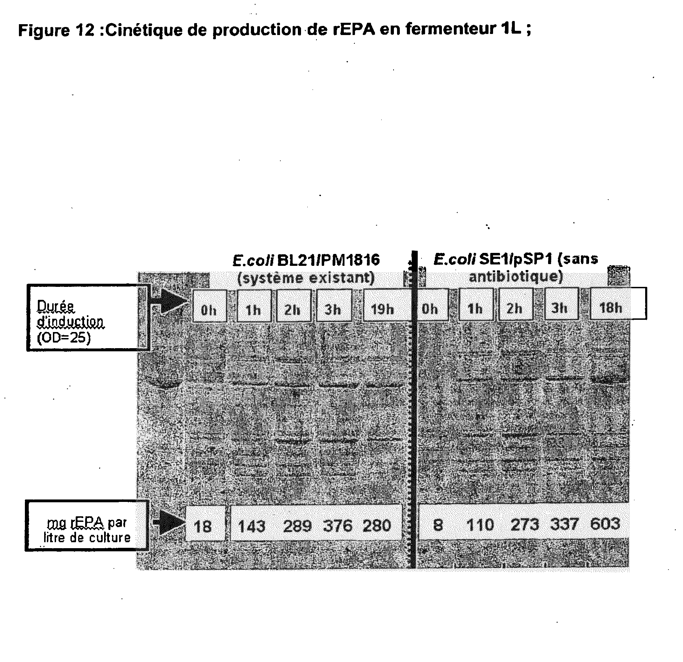

Le gel de la figure 12 documente la présence de rEPA en cours d'induction.

Avec le nouveau système, l'induction peut être maintenue pendant 24 h sans perte significative de plasmide, même si l'induction par l'IPTG s'effectue en début de culture. Dans ces mêmes conditions d'induction rapide, seulement 5% des cellules retiennent le plasmide avec le système basé sur la sélection Km comme le montre le tableau ci-dessous :

| | **Induction à A₆₀₀ = 1** | | **Induction à A₆₀₀ = 25** | |
|---|---|---|---|---|
| | E. coli BL21 / PM1816 | E. coli SE 1 / pSP1 | E. coli BL21 / PM1816 | E. coli SE 1 / pSP1 |
| A₆₀₀ | 65 | 49 | 41 | 47 |
| % P⁺ | 5% | 100% | 90% | 98% |
| rEPA (mg/litre) | 36 | 350 | 280 | 603 |

Productivité de rEPA en fermenteur 1 L après 24h de culture.
- % P+ mesure le pourcentage de cellules contenant le plasmide parmi les cellules totales viables.
- la quantité de rEPA est évaluée par ELISA comme cela est décrit dans les exemples précédents.

### Evaluation en fermenteur 30L:

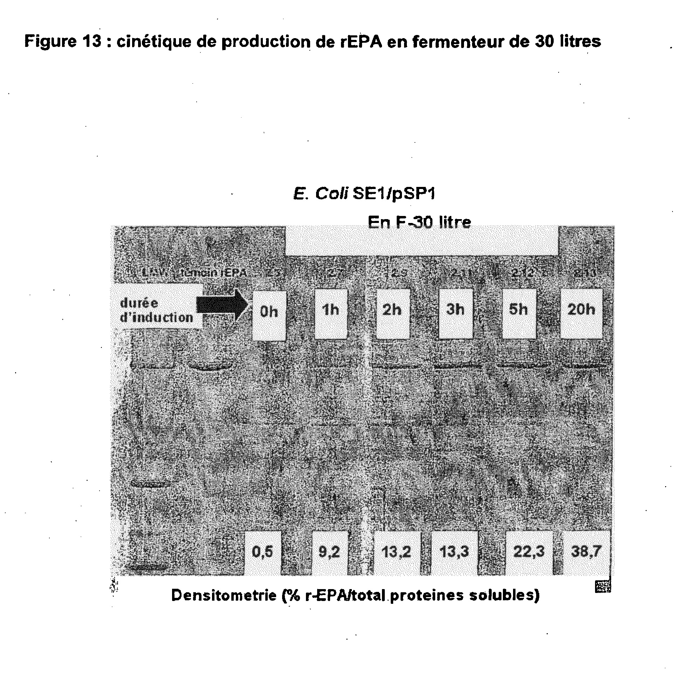

La nouvelle construction a été ensuite testée à l'échelle standard de production des lots cliniques, soit 30L. Les paramètres de culture restent les mêmes que ceux décrits lors des précédentes cultures. Le volume de milieu de culture dans le fermenteur est de 20 litres. La cinétique de production de rEPA est donnée à la figure 13.

Nous avons évalué le nouveau vecteur pour la production d'une protéine recombinante. Ce système s'affranchit de la présence de gène de résistance aux antibiotiques et n'a donc plus recourt à l'utilisation d'antibiotique quelque soit l'étape de la culture. L'évaluation à l'échelle pilote en fermenteur 30L a montré que le plasmide est maintenu tout au long de la culture, sans qu'aucune perte ne soit observée.

La productivité en protéine d'intérêt obtenue supérieure à celle observée avec le système classique utilisant comme marqueur de sélection un gène de résistance à un antibiotique.

### Exemple 4 Analyse de l'intérêt du fragment Cer

Pour évaluer la contribution du fragment Cer à l'augmentation de la production observée, différents vecteurs ont été construits.

Le vecteur pM1800 a été amplifié par PCR afin d'éliminer le gène de résistance à la kanamycine et de créer les sites de restriction Pstl et Acc65I.

La cassette SEQ ID NO 8 a été construite par PCR combinées à partir du vecteur pM1800.

Dans un premier temps le promoteur mob et la partie 5' du gène ccdA ont été amplifié à partir du plasmide pStaby (Delphi gentic). L'amorce PCR apporte un fragment de la partie 3' du gène kanamycine. Cette PCR est nommée C1.

Une deuxième PCR permet d'amplifier la partie 3' du gène ccdA. L'amorce PCR apportant un fragment de la partie 5' du gène kanamycine. Cette amplification est nommée C2.

La troisième PCR concerne l'amplification du gène Kanamycine à partir du pM1800. L'amorce 5' apporte un fragment de la partie 5' du gène ccdA. L'amorce 3' apporte un fragment de la partie 3' du gène ccdA. Cette PCR est nommée C3.

La PCR C1 est combinée à la PCR C2 et le produit est ensuite combiné à la PCR C3. Le fragment obtenu est digéré par les enzymes Pstl et Acc65I et cloné dans le vecteur pM1800 digéré par ces mêmes enzymes.

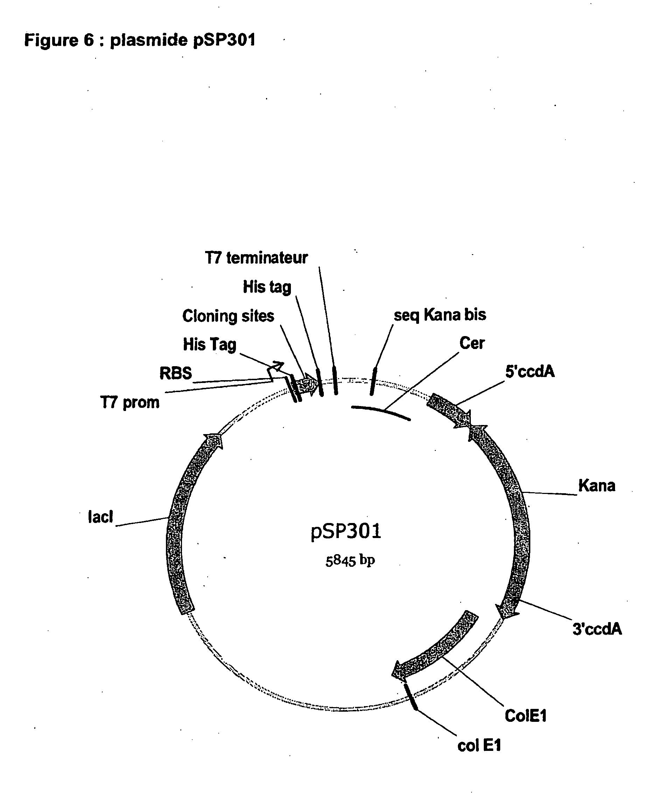

La ligation des deux fragments a été réalisée avec l'enzyme : T4 DNA Ligase New England BioLabs. (400U/µL) pendant la durée une nuit à 16°C. Le nouveau vecteur crée est nommé pSP301. Une représentation de ce vecteur est donnée à la figure 6.

Une version de ce vecteur sans le fragment cer a été construite. Le vecteur pSP301 a été digéré par les enzymes Pacl et Ascl, délétant ainsi le fragment cer. Les extrémités cohésives ainsi libérées ont été traitées avec l'exonuclease « Mung Bean ». (New England BioLabs) utilisée à 10U/µl, à 30°C pendant 1 h. La re-ligation du vecteur aux extrémités franches a été réalisée avec l'enzyme : T4 DNA Ligase New England BioLabs. (400U/µL) pendant une nuit à 16°C

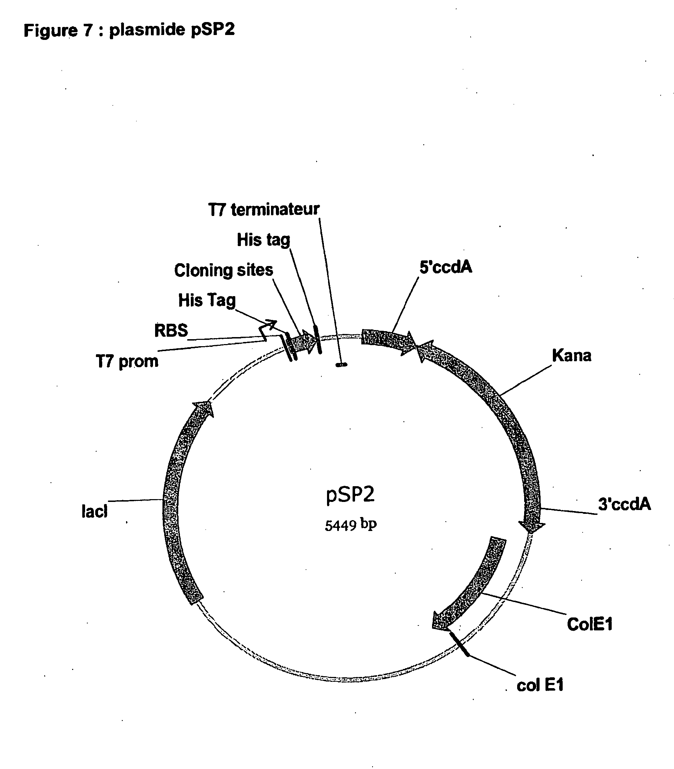

Le nouveau vecteur ainsi construit est nommé pSP2. Une représentation schématique du plasmide pSP2 est donnée à la figure 7

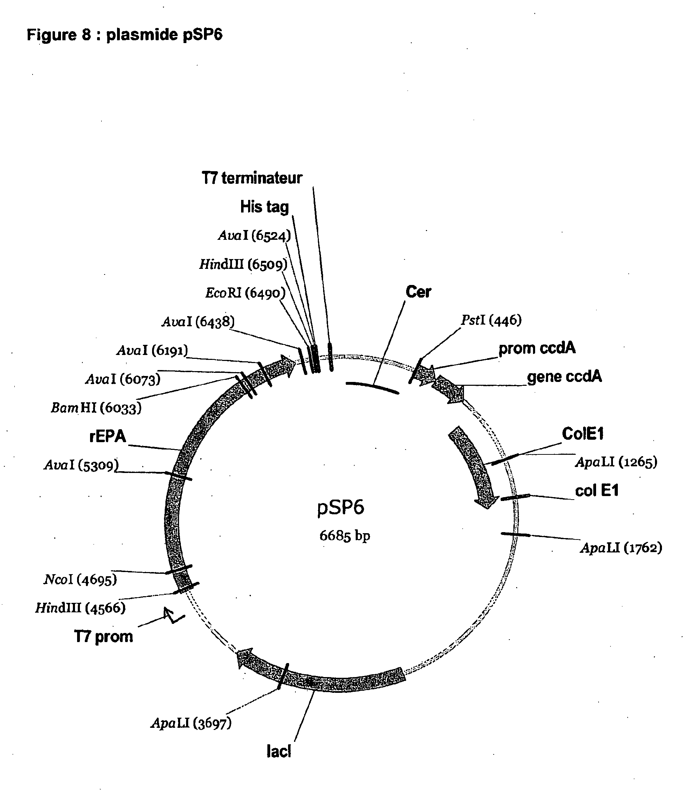

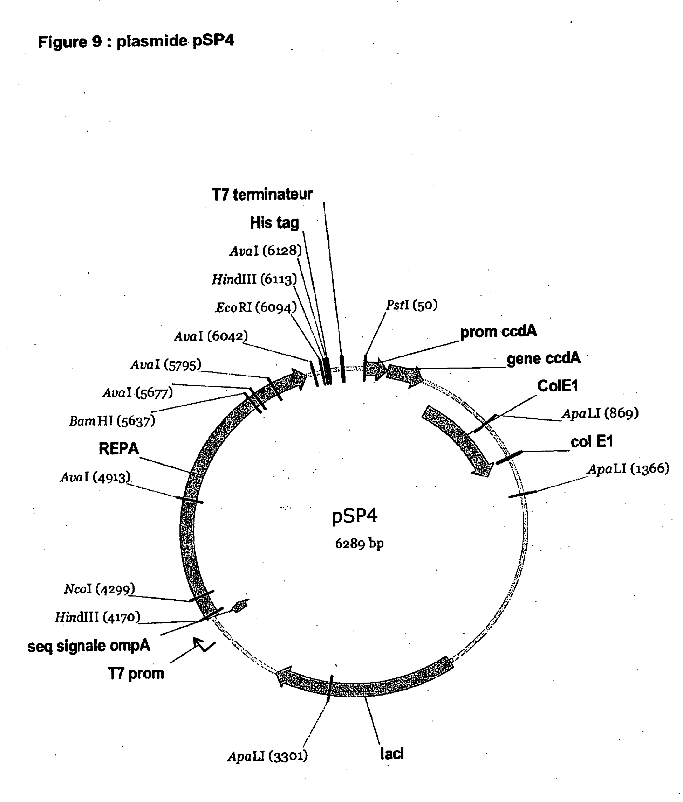

La cassette SEQ ID NO9 (contenant RBS-Omp A-rEPA, 2024pb), isolée du plasmide pM1816, a été clonée dans les vecteurs pSP301 et pSP2 entre les sites XbaI et EcoRI. Le gène kanamycine a ensuite été excisé de ces vecteurs par recombinaison homologue comme décrit dans l'exemple 4. Les plasmides obtenus sont nommés respectivement, pSP6 et pSP4 dont une représentation schématique est donnée aux figures 8 et 9.

Les vecteurs pSP6 et pSP4 ont été transformés dans la souche SE1, et le vecteur pM1816 dans la souche BL21λDE3. Un essai d'expression comparatif a été réalisé à partir de ces 3 vecteurs. Les mêmes conditions opératoires que celles de l'exemple 2 ont été utilisées.

Les meilleurs résultats de production de rEPA ont été obtenus avec les constructions contenant le fragment cer.

Afin de conforter les premiers résultats obtenus, le même type de travail a été réalisé avec un second antigène : la protéine AlpA *d'Helicobacter pylori* Les vecteurs suivants ont été construits

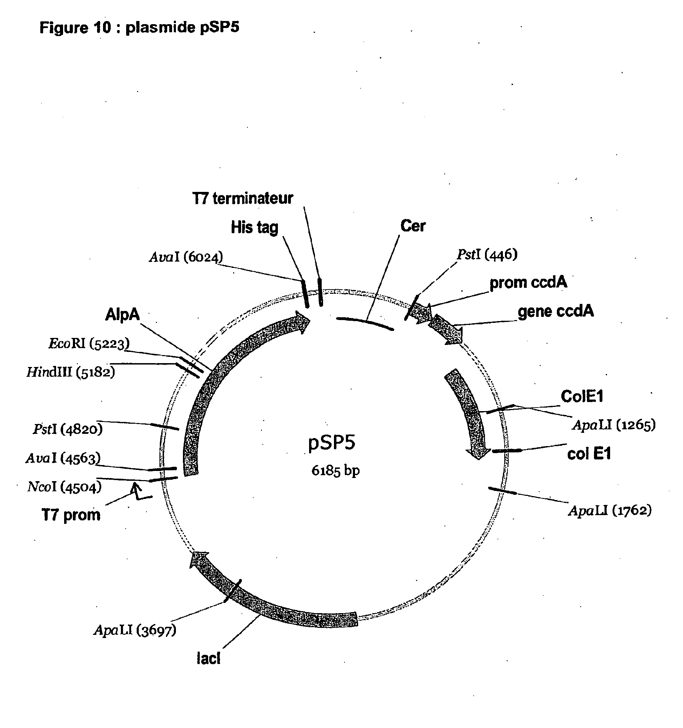

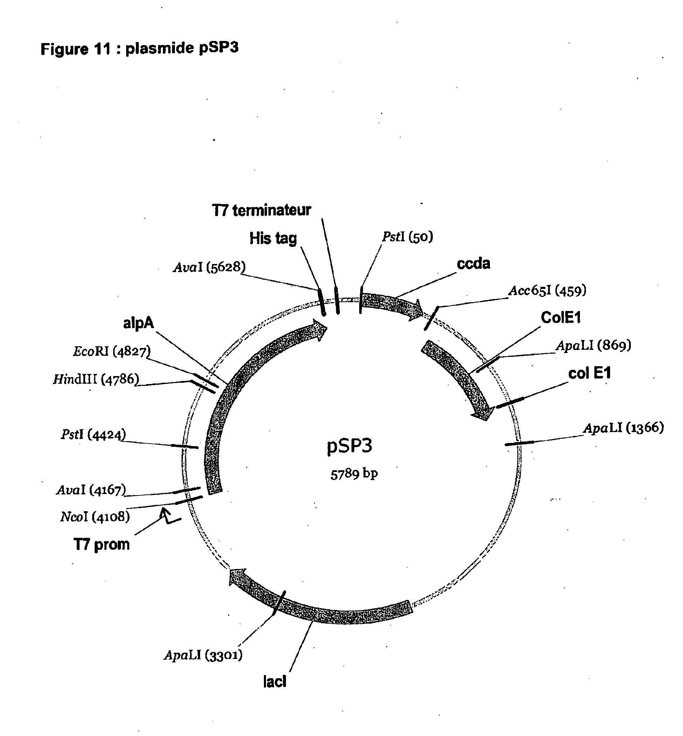

Le gène de la protéine AlpA (SEQ ID No 4) a été cloné dans les vecteur pSP301 et pSP2 (précédemment décrits) entre les sites Ncol/Xabal. Le gène kanamycine de ces nouveaux plasmides est excisé par recombinaison homologue, comme décrit dans l'exemple 4, créant ainsi respectivement les vecteurs pSP5 et pSP3. Une représentation schématique de ces vecteurs est donnée aux figures 10 et 11.

Les vecteurs pSP5 et pSP3 ont été transformés dans la souche SE1, et le vecteur pMH.P3.1 dans la souche BL21λDE3. Un essai d'expression comparatif a été réalisé à partir de ces 3 vecteurs. Les mêmes conditions opératoires que celles de l'exemple 2 ont été utilisées.

Les résultats obtenus montrent que la quantité de protéine d'intérêt produite (% exprimé par rapport aux protéines totales) est supérieure avec les vecteurs « sans antibio » contenant la séquence Cer. L'analyse densitométrique des bandes obtenues par électrophorèse en SDS PAGE indique clairement une diminution d'expression en absence du fragment Cer.

La comparaison des différentes constructions montre qu'un vecteur « sans antibiotique » + cer conduit à des productivités nettement supérieures à celles obtenues avec un vecteur « antibiotique » +cer, lequel conduit à une productivité supérieure à celle obtenue avec un vecteur « antibiotique » sans Cer.

D'autre part, on observe que le vecteur « sans antibiotique » + cer conduit à une augmentation de production de la protéine d'intérêt d'au moins 10% par rapport à un système « antibiotique » sans cer.

### Exemple 4 Suppression du gène kanamycine par recombinaison homologue

Les vecteurs pSP301 ou pSP2 sont digérés par l'enzyme ClaI

2µg d'ADN plasmidique sont digérés avec 6U d'enzyme ClaI, 1h à 37°C. Puis, 200 ng de cette digestion est transformée avec 100µl de bactéries CYS21(Delphigenetics) électrocompétentes. La totalité de la transformation est étalée sur boite LB.

Les clones obtenus sont ensuite analysés en minipréparation d'ADN par digestion enzymatique.

L'analyse de clones obtenus après transfection de bactéries CYS21 électrocompétentes par l'ADN linéarisé montre que toutes les colonies obtenues contiennent un plasmide recombiné. Le séquençage de 2 clones pris au hasard a permis, en outre, de confirmer que l'assemblage du gène ccdA était conforme au résultat attendu.

Cette recombinaison homologue a été réalisée avec les plasmides pSP3, pSP5 et pSP4, pSP6.

Le procédé de recombinaison homologue apporte une sélection positive des clones recombinés dans la mesure où le gène ccdA ne peut être fonctionnel qu'après élimination du gène de résistance à la Kanamycine. Nous avons ainsi la certitude que les bactéries transformées ont perdu le gène de résistance à la Kanamycine, ce qui n'est pas le cas lorsque l'on utilise le système basé sur une étape de digestion enzymatique suivi d'une re-ligation. Cette méthode a donc pour effet de s'affranchir totalement d'une analyse supplémentaire visant à documenter l'absence du gène de résistance à la Kanmycine. Cette analyse supplémentaire serait classiquement réalisée sur une préparation d'ADN plasmidique analysée par cartographie de restriction ou encore à l'aide du méthode d'amplification spécifique par PCR.

## Revendications

1. Vecteur auto réplicatif dépourvu de gène de résistance à un antibiotique comprenant:
- une séquence codant pour la protéine ccdA liée de façon fonctionnelle à un premier promoteur,
- la séquence du locus Cer et
- une séquence hétérologue, liée de façon fonctionnelle à un deuxième promoteur.

2. Vecteur selon la revendication 1 dans lequel le premier promoteur est le promoteur constitutif mob.

3. Vecteur selon la revendication 1 ou 2 dans lequel le deuxième promoteur est un promoteur inductible.

4. Vecteur selon la 3 dans lequel le deuxième promoteur est le promoteur T7.

5. Vecteur selon l'une quelconque des revendications 1 à 4 dans lequel la séquence hétérologue code pour un antigène vaccinal.

6. Vecteur selon la revendication 5 dans lequel la séquence hétérologue code pour la protéine rEPA.

7. Vecteur selon l'une quelconque des revendications 1 à 4 dans lequel la séquence hétérologue code pour une séquence utilisable dans le cadre d'une thérapie génique.

8. Vecteur selon l'une quelconque des revendications 1 à 4 dans lequel la séquence hétérologue code pour une enzyme.

9. Cellule procaryote exprimant la protéine ccdB contenant un vecteur d'expression tel que défini dans l'une quelconque des revendications 1 à 8.

10. Cellule procaryote selon la revendication 9 correspondant à une cellule d'E.coli

11. Procédé de production d'une protéine hétérologue comprenant les étapes de
(a) ensemencement d'un milieu de culture approprié avec des cellules telles que définies dans la revendication 9 ou 10 et contenant un vecteur tel que défini dans l'une quelconque des revendications1 à 8,
(b) culture en fermenteur des cellules ainsi transformée en l'absence d'antibiotique et
(c) récupération de la protéine hétérologue produite au cours de l'étape (b) à partir du surnageant ou du culot cellulaire.

12. Procédé de production d'une protéine hétérologue selon la revendication 11 dans laquelle la protéine hétérologue est la rEPA.

13. Procédé de production d'un vecteur auto réplicatif tel que défini dans l'une quelconque des revendications 1 à 8 comprenant les étapes de :
(a) ensemencement d'un milieu de culture approprié avec des cellules procaryotes exprimant la protéine ccdB et contenant un vecteur tel que défini ci-dessus,
(b) culture en fermenteur de la cellule ainsi transformée en l'absence d'antibiotique et
(c) récupération du vecteur produit au cours de l'étape (b).

14. Procédé de production d'un vecteur d'expression auto réplicatif dépourvu de gène de résistance à un antibiotique tel que défini dans l'une quelconque des revendications 1 à 8 comprenant les étapes de :
(a) construction d'un vecteur auto réplicatif, **caractérisé en ce que** la séquence codant pour la ccdA est constituée de deux séquences 5' et 3' chevauchantes situées de part et d'autre d'un gène de résistance à un antibiotique dont la présence rend le gène ccdA non fonctionnel,
(b) linéarisation dudit vecteur par utilisation d'une enzyme de restriction coupant le vecteur uniquement entre les séquences 5' et 3'
(c) transformation d'une cellule procaryote exprimant la protéine ccdB en présence du vecteur linéarisé, et re-circularisation du vecteur par recombinaison homologue **caractérisé en ce que** la séquence ccdA devient fonctionnelle par élimination du gène de résistance à l'antibiotique, et
(d) récupération des cellules procaryotes comprenant le vecteur auto réplicatif tel que défini selon l'une des revendications 1 à 8 dépourvu de gène de résistance à un antibiotique et contenant une séquence ccdA fonctionnelle.

## Patentansprüche

1. Selbstreplizierender Vektor ohne Antibiotikumresistenzgen, umfassend:
- eine für das Protein ccdA codierende Sequenz, die funktionell mit einem ersten Promotor verknüpft ist,
- die Sequenz des Cer-Locus und
- eine heterologe Sequenz, die funktionell mit einem zweiten Promotor verknüpft ist.

2. Vektor nach Anspruch 1, wobei der erste Promotor der konstitutive mob-Promotor ist.

3. Vektor nach Anspruch 1 oder 2, wobei der zweite Promotor ein induzierbarer Promotor ist.

4. Vektor nach Anspruch 3, wobei der zweite Promotor der T7-Promotor ist.

5. Vektor nach einem der Ansprüche 1 bis 4, wobei die heterologe Sequenz für ein Impfstoffantigen codiert.

6. Vektor nach Anspruch 5, wobei die heterologe Sequenz für das Protein rEPA codiert.

7. Vektor nach einem der Ansprüche 1 bis 4, wobei die heterologe Sequenz für eine Sequenz codiert, die im Rahmen einer Gentherapie verwendbar ist.

8. Vektor nach einem der Ansprüche 1 bis 4, wobei die heterologe Sequenz für ein Enzym codiert.

9. Prokaryotische Zelle, die das Protein ccdB exprimiert und einen Expressionsvektor nach einem der Ansprüche 1 bis 8 enthält.

10. Prokaryotische Zelle nach Anspruch 9, die einer E. coli-Zelle entspricht.

11. Verfahren zur Herstellung eines heterologen Proteins, das folgende Schritte umfasst:
(a) Beimpfen eines geeigneten Kulturmediums mit Zellen nach Anspruch 9 oder 10, die einen Vektor nach einem der Ansprüche 1 bis 8 enthalten,
(b) Fermenterzüchtung der so transformierten Zellen in Abwesenheit eines Antibiotikums und
(c) Gewinnen des im Verlauf von Schritt (b) hergestellten heterologen Proteins aus dem Überstand oder dem Zellsediment.

12. Verfahren zur Herstellung eines heterologen Proteins nach Anspruch 11, wobei es sich bei dem heterologen Protein um rEPA handelt.

13. Verfahren zur Herstellung eines selbstreplizierenden Vektors nach einem der Ansprüche 1 bis 8, das folgende Schritte umfasst:
(a) Beimpfen eines geeigneten Kulturmediums mit prokaryotischen Zellen, die das Protein ccdB exprimieren und einen Vektor, wie vorstehend definiert, enthalten,
(b) Fermenterzüchtung der so transformierten Zelle in Abwesenheit eines Antibiotikums und
(c) Gewinnen des im Verlauf von Schritt (b) hergestellten Vektors.

14. Verfahren zur Herstellung eines selbstreplizierenden Expressionsvektors ohne Antibiotikumresistenzgen nach einem der Ansprüche 1 bis 8, das folgende Schritte umfasst:
(a) Konstruktion eines selbstreplizierenden Vektors, **dadurch gekennzeichnet, dass** die für ccdA codierende Sequenz aus zwei überlappenden 5'- und 3'-Sequenzen besteht, die sich auf beiden Seiten eines Antibiotikumresistenzgens befinden, dessen Vorhandensein das ccdA-Gen nicht-funktionell macht,
(b) Linearisierung des Vektors unter Verwendung eines Restriktionsenzyms, das den Vektor einmal zwischen den 5'- und 3'-Sequenzen spaltet,
(c) Transformation einer das Protein ccdB exprimierenden prokaryotischen Zelle in Anwesenheit des linearisierten Vektors und Rezirkularisierung des Vektors durch homologe Rekombination, **dadurch gekennzeichnet, dass** die ccdA-Sequenz durch die Elimination des Antibiotikumresistenzgens funktionell wird, und
(d) Gewinnen der prokaryotischen Zellen, die den selbstreplizierenden Vektor nach einem der Ansprüche 1 bis 8 ohne Antibiotikumresistenzgen umfassen und eine funktionelle ccdA-Sequenz enthalten.

## Claims

1. Self-replicating vector devoid of any antibiotic-resistance gene, comprising:
- a sequence encoding the ccdA protein functionally linked to a first promoter,
- the sequence of the Cer locus, and
- a heterologous sequence functionally linked to a second promoter.

2. Vector according to Claim 1, in which the first promoter is the mob constitutive promoter.

3. Vector according to Claim 1 or 2, in which the second promoter is an inducible promoter.

4. Vector according to Claim 3, in which the second promoter is the T7 promoter.

5. Vector according to any one of Claims 1 to 4, in which the heterologous sequence encodes a vaccine antigen.

6. Vector according to Claim 5, in which the heterologous sequence encodes the rEPA protein.

7. Vector according to any one of Claims 1 to 4, in which the heterologous sequence encodes a sequence that can be used in the context of gene therapy.

8. Vector according to any one of Claims 1 to 4, in which the heterologous sequence encodes an enzyme.

9. Prokaryotic cell expressing the ccdB protein, containing an expression vector as defined in any one of Claims 1 to 8.

10. Prokaryotic cell according to Claim 9 corresponding to an *E. coli* cell.

11. Method for producing a heterologous protein, comprising the steps of:
(a) inoculating an appropriate culture medium with cells as defined in Claim 9 or 10 and containing a vector as defined in any one of Claims 1 to 8,
(b) fermenter culturing the cells thus transformed in the absence of antibiotic, and
(c) recovering the heterologous protein produced during step (b) from the supernatant or from the cell pellet.

12. Method, according to Claim 11, for producing a heterologous protein, in which the heterologous protein is rEPA.

13. Method for producing a self-replicating vector as defined in any one of Claims 1 to 8, comprising the steps of:
(a) inoculating an appropriate culture medium with prokaryotic cells expressing the ccdB protein and containing a vector as defined above,
(b) fermenter culturing the cell thus transformed in the absence of antibiotic, and
(c) recovering the vector produced during step (b).

14. Method for producing a self-replicating expression vector devoid of any antibiotic-resistance gene as defined in any one of Claims 1 to 8, comprising the steps of:
(a) constructing a self-replicating vector, **characterized in that** the sequence encoding ccdA consists of two overlapping 5' and 3' sequences located on either side of an antibiotic-resistance gene, the presence of which renders the ccdA gene non-functional,
(b) linearizing said vector by using a restriction enzyme which cleaves the vector only between the 5' and 3' sequences,
(c) transforming a prokaryotic cell expressing the ccdB protein in the presence of the linearized vector, and recircularizing the vector by homologous recombination **characterized in that** the ccdA sequence becomes functional through elimination of the antibiotic-resistance gene, and
(d) recovering the prokaryotic cells comprising the self-replicating vector as defined in one of Claims 1 to 8 devoid of any antibiotic-resistance gene and comprising a functional ccdA sequence.


### SEQUENCE LISTING

<110> sanofi Pasteur
<120> vecteur auto réplicatif
<130> PM 0804 FR
<160> 9
<170> PatentIn version 3.3
<210> 1
   <211> 284
   <212> DNA
   <213> E-coli
<400> 1
   ccggaaatac aggaacgcac gctggatggc ccttcgctgg gatggtgaaa ccatgaaaaa     60
   tggcagcttc agtggattaa gtgggggtaa tgtggcctgt accctctggt tgcataggta    120
   ttcatacggt taaaatttat caggcgcgat cgcggcagtt tttcgggtgg tttgttgcca    180
   tttttacctg tctgctgccg tgatcgcgct gaacgcgttt tagcggtgcg tacaattaag    240
   ggattatggt aaatccactt actgtctgcc ctcgtagcca tcga                     284
<210> 2
   <211> 31
   <212> DNA
   <213> Artificial
<220>
   <223> Primer
<400> 2
   tccttgcggc cgcacatcac cgatggggaa g 31
<210> 3
   <211> 26
   <212> DNA
   <213> Artificial
<220>
   <223> Primer
<400> 3
   ctgaattctc accagtccct gttctc 26
<210> 4
   <211> 1512
   <212> DNA
   <213> Helicobacter pilory
<400> 4
   atggctagca taagttatgc cgaagatgat ggagggtttt tcaccgtcgg ttatcagctc 60
   gggcaggtca tgcaagatgt ccaaaaccca ggcggcgcta aaagcgacga actcgccaga 120
   gagcttaacg ctgatgtaac gaacaacatt ttaaacaaca acaccggagg caatgtcgca 180
   ggggcgttga gtaacgcttt ctcccaatac ctttattcgc ttttaggggc ttacccaaca 240
   aaactcaatg gtagcgatgt gtctgcgaac gctcttttaa gtggtgcggt aggcagtggg 300
   acttgcacgg ctgcagggac ggccggtggc acatctctta acactcaaag cgcttgcacc 360
   gctgcgggat attactggct ccctagcttg actgacagga ttttaagcac gatcggcagc 420
   cagactaact acggcacgaa caccaatttc cccaacatgc aacaacagct cacctacttg 480
   aatgcgggga atgtgttttt taatgcgatg aataaggctt tagggaagaa tgggactgct 540
   actactagtg gcactagcag tgcaactggt tcagatggtc aaacttactc cacacaagcc 600
   atccaatacc ttcaaaacca acaaaatatc ttaaataacg cagcgaactt gctcaagcaa 660
   gatgaattgc tcctagaagc tttcaactct gccgtagctg ctaacattgg gaataaggaa 720
   ttcaattcag ccgcttttac aggtttggtg caaggcatta ttgatcaatc tcaattggtt 780
   tataacgagc tcactaaaaa caccattagc gggagcgcgg ttaatggcgc tgggataaac 840
   tccaaccaag ctaatgctgt gcaagggcgt gccagtcagc tccctaacgc tctttataac 900
   gcgcaagtaa ctttggataa aatcaatgcg ctcaataatc aggtgagaag catgccttac 960
   ttgccccaat tcagagccgg gaacagccgt gcaacgaata ttttaaacgg gttttacacc 1020
   aaaataggct ataagcaatt cttcgggaag aaaaggaata tcggtttgcg ctattatggt 1080
   ttcttttctt ataacggagc gagcgtgggc tttagatcca ctcaaaataa tgtagggtta 1140
   tacacttatg gggtggggac tgatgtgttg tataacatct ttagccgctc ctatcaaaac 1200
   cgctctgtgg atatgggctt ttttagcggc atccaattag ccggtgagac tttccaatcc 1260
   acgctcagag atgaccccaa tgtgaaattg catgggaaaa tcaataacac gcacttccag 1320
   ttcctctttg acttcggtat gaggatgaac ttcggtaagt tggacgggaa atccaaccgc 1380
   cacaaccagc acacggtgga atttggcgta gtggtgccta cgatttataa cacttattac 1440
   aaatcagcag ggactaccgt gaagtatttc cgtccttata gcgtttattg gtcttatggg 1500
   tattcattct aa 1512
<210> 5
   <211> 1184
   <212> DNA
   <213> virus de la grippe
<400> 5
   atgaaagcaa aactactggt cctgttatgc acatttacag ctacatatgc agacacaata 60
   tgtataggct accatgccaa caactcaacc gacactgttg acacagtact tgagaagaat 120
   gtgacagtga cacactctgt caacctgctt gaggacagtc acaatggaaa actatgtcta 180
   ttaaaaggaa tagccccact acaattgggt aattgcagcg ttgccgggtg gatcttagga 240
   aacccagaat gcgaattact gatttccaag gaatcatggt cctacattgt agaaaaacca 300
   aatcctgaga atggaacatg ttacccaggg tatttcgccg actatgagga actgagggag 360
   caattgagtt cagtatcttc atttgagagg ttcgaaatat tccccaaaga aagctcatgg 420
   cccaaccaca ccgtaaccgg agtatcagca tcatgctccc ataatgggaa aagcagtttt 480
   tacaaaaatt tgctatggct gacggggaag aatggtttgt acccaaacct gagcaagtcc 540
   tatgcaaaca acaaagagaa agaagtcctt gtactatggg gtgttcatca cccgcctaac 600
   ataggtgacc aaagggccct ctatcataca gaaaatgctt atgtctctgt agtgtcttca 660
   cattatagca gaaaattcac cccagaaata gccaaaagac ccaaagtaag agatcaagaa 720
   ggaagaatca actactactg gactctgctt gaacccgggg atacaataat atttgaggca 780
   aatggaaatc taatagcgcc aagatatgct ttcgcactga gtagaggctt tggatcagga 840
   atcatcacct caaatgcacc aatggatgaa tgtgatgcga agtgccaaac acctcaggga 900
   gctataaaca gcagtcttcc tttccagaat gtacacccag tcacaatagg agagtgtcca 960
   aagtatgtca ggagtgcaaa attaaggatg gttacaggac taaggaacat cccatccatt 1020
   caatccagag gtttgtttgg agccattgcc ggtttcattg aaggggggtg gactggaatg 1080
   gtagatggtt ggtatggtta tcatcatcag aatgagcaag gctctggcta tgctgcagat 1140
   caaaaaagca cacaaaatgc cattaatggg attacaaaca agtg 1184
<210> 6
   <211> 4443
   <212> DNA
   <213> Humaine
<400> 6
   atgcagaggt cgcctctgga aaaggccagc gttgtctcca aacttttttt cagctggacc 60
   agaccaattt tgaggaaagg atacagacag cgcctggaat tgtcagacat ataccaaatc 120
   ccttctgttg attctgctga caatctatct gaaaaattgg aaagagaatg ggatagagag 180
   ctggcttcaa agaaaaatcc taaactcatt aatgcccttc ggcgatgttt tttctggaga 240
   tttatgttct atggaatctt tttatattta ggggaagtca ccaaagcagt acagcctctc 300
   ttactgggaa gaatcatagc ttcctatgac ccggataaca aggaggaacg ctctatcgcg 360
   atttatctag gcataggctt atgccttctc tttattgtga ggacactgct cctacaccca 420
   gccatttttg gccttcatca cattggaatg cagatgagaa tagctatgtt tagtttgatt 480
   tataagaaga ctttaaagct gtcaagccgt gttctagata aaataagtat tggacaactt 540
   gttagtctcc tttccaacaa cctgaacaaa tttgatgaag gacttgcatt ggcacatttc 600
   gtgtggatcg ctcctttgca agtggcactc ctcatggggc taatctggga gttgttacag 660
   gcgtctgcct tctgtggact tggtttcctg atagtccttg ccctttttca ggctgggcta 720
   gggagaatga tgatgaagta cagagatcag agagctggga agatcagtga aagacttgtg 780
   attacctcag aaatgattga aaatatccaa tctgttaagg catactgctg ggaagaagca 840
   atggaaaaaa tgattgaaaa cttaagacaa acagaactga aactgactcg gaaggcagcc 900
   tatgtgagat acttcaatag ctcagccttc ttcttctcag ggttctttgt ggtgttttta 960
   tctgtgcttc cctatgcact aatcaaagga atcatcctcc ggaaaatatt caccaccatc 1020
   tcattctgca ttgttctgcg catggcggtc actcggcaat ttccctgggc tgtacaaaca 1080
   tggtatgact ctcttggagc aataaacaaa atacaggatt tcttacaaaa gcaagaatat 1140
   aagacattgg aatataactt aacgactaca gaagtagtga tggagaatgt aacagccttc 1200
   tgggaggagg gatttgggga attatttgag aaagcaaaac aaaacaataa caatagaaaa 1260
   acttctaatg gtgatgacag cctcttcttc agtaatttct cacttcttgg tactcctgtc 1320
   ctgaaagata ttaatttcaa gatagaaaga ggacagttgt tggcggttgc tggatccact 1380
   ggagcaggca agacttcact tctaatggtg attatgggag aactggagcc ttcagagggt 1440
   aaaattaagc acagtggaag aatttcattc tgttctcagt tttcctggat tatgcctggc 1500
   accattaaag aaaatatcat ctttggtgtt tcctatgatg aatatagata cagaagcgtc 1560
   atcaaagcat gccaactaga agaggacatc tccaagtttg cagagaaaga caatatagtt 1620
   cttggagaag gtggaatcac actgagtgga ggtcaacgag caagaatttc tttagcaaga 1680
   gcagtataca aagatgctga tttgtattta ttagactctc cttttggata cctagatgtt 1740
   ttaacagaaa aagaaatatt tgaaagctgt gtctgtaaac tgatggctaa caaaactagg 1800
   attttggtca cttctaaaat ggaacattta aagaaagctg acaaaatatt aattttgcat 1860
   gaaggtagca gctattttta tgggacattt tcagaactcc aaaatctaca gccagacttt 1920
   agctcaaaac tcatgggatg tgattctttc gaccaattta gtgcagaaag aagaaattca 1980
   atcctaactg agaccttaca ccgtttctca ttagaaggag atgctcctgt ctcctggaca 2040
   gaaacaaaaa aacaatcttt taaacagact ggagagtttg gggaaaaaag gaagaattct 2100
   attctcaatc caatcaactc tatacgaaaa ttttccattg tgcaaaagac tcccttacaa 2160
   atgaatggca tcgaagagga ttctgatgag cctttagaga gaaggctgtc cttagtacca 2220
   gattctgagc agggagaggc gatactgcct cgcatcagcg tgatcagcac tggccccacg 2280
   cttcaggcac gaaggaggca gtctgtcctg aacctgatga cacactcagt taaccaaggt 2340
   cagaacattc accgaaagac aacagcatcc acacgaaaag tgtcactggc ccctcaggca 2400
   aacttgactg aactggatat atattcaaga aggttatctc aagaaactgg cttggaaata 2460
   agtgaagaaa ttaacgaaga agacttaaag gagtgctttt ttgatgatat ggagagcata 2520
   ccagcagtga ctacatggaa cacatacctt cgatatatta ctgtccacaa gagcttaatt 2580
   tttgtgctaa tttggtgctt agtaattttt ctggcagagg tggctgcttc tttggttgtg 2640
   ctgtggctcc ttggaaacac tcctcttcaa gacaaaggga atagtactca tagtagaaat 2700
   aacagctatg cagtgattat caccagcacc agttcgtatt atgtgtttta catttacgtg 2760
   ggagtagccg acactttgct tgctatggga ttcttcagag gtctaccact ggtgcatact 2820
   ctaatcacag tgtcgaaaat tttacaccac aaaatgttac attctgttct tcaagcacct 2880
   atgtcaaccc tcaacacgtt gaaagcaggt gggattctta atagattctc caaagatata 2940
   gcaattttgg atgaccttct gcctcttacc atatttgact tcatccagtt gttattaatt 3000
   gtgattggag ctatagcagt tgtcgcagtt ttacaaccct acatctttgt tgcaacagtg 3060
   ccagtgatag tggcttttat tatgttgaga gcatatttcc tccaaacctc acagcaactc 3120
   aaacaactgg aatctgaagg caggagtcca attttcactc atcttgttac aagcttaaaa 3180
   ggactatgga cacttcgtgc cttcggacgg cagccttact ttgaaactct gttccacaaa 3240
   gctctgaatt tacatactgc caactggttc ttgtacctgt caacactgcg ctggttccaa 3300
   atgagaatag aaatgatttt tgtcatcttc ttcattgctg ttaccttcat ttccatttta 3360
   acaacaggag aaggagaagg aagagttggt attatcctga ctttagccat gaatatcatg 3420
   agtacattgc agtgggctgt aaactccagc atagatgtgg atagcttgat gcgatctgtg 3480
   agccgagtct ttaagttcat tgacatgcca acagaaggta aacctaccaa gtcaaccaaa 3540
   ccatacaaga atggccaact ctcgaaagtt atgattattg agaattcaca cgtgaagaaa 3600
   gatgacatct ggccctcagg gggccaaatg actgtcaaag atctcacagc aaaatacaca 3660
   gaaggtggaa atgccatatt agagaacatt tccttctcaa taagtcctgg ccagagggtg 3720
   ggcctcttgg gaagaactgg atcagggaag agtactttgt tatcagcttt tttgagacta 3780
   ctgaacactg aaggagaaat ccagatcgat ggtgtgtctt gggattcaat aactttgcaa 3840
   cagtggagga aagcctttgg agtgatacca cagaaagtat ttattttttc tggaacattt 3900
   agaaaaaact tggatcccta tgaacagtgg agtgatcaag aaatatggaa agttgcagat 3960
   gaggttgggc tcagatctgt gatagaacag tttcctggga agcttgactt tgtccttgtg 4020
   gatgggggct gtgtcctaag ccatggccac aagcagttga tgtgcttggc tagatctgtt 4080
   ctcagtaagg cgaagatctt gctgcttgat gaacccagtg ctcatttgga tccagtaaca 4140
   taccaaataa ttagaagaac tctaaaacaa gcatttgctg attgcacagt aattctctgt 4200
   gaacacagga tagaagcaat gctggaatgc caacaatttt tggtcataga agagaacaaa 4260
   gtgcggcagt acgattccat ccagaaactg ctgaacgaga ggagcctctt ccggcaagcc 4320
   atcagcccct ccgacagggt gaagctcttt ccccaccgga actcaagcaa gtgcaagtct 4380
   aagccccaga ttgctgctct gaaagaggag acagaagaag aggtgcaaga tacaaggctt 4440
   tag 4443
<210> 7
   <211> 357
   <212> DNA
   <213> Bactérienne - Promoteur mob + gène ccdA
<400> 7
   cgccgcagcc gccgtaggcg gtcacgactt tgcgaagcaa agtctagtga gtatactcaa 60
   gcattgagtg gcccgccgga ggcaccgcct tgcgctgccc ccgtcgagcc ggttggtcac 120
   caaaagggag gggcaggcat gaagcagcgt attacagtga cagttgacag cgacagctat 180
   cagttgctca aggcatatga tgtcaatatc tccggtctgg taagcacaac catgcagaat 240
   gaagcccgtc gtctgcgtgc cgaacgctgg aaagcggaaa atcaggaagg gatggctgag 300
   gtcgcccggt ttattgaaat gaacggctct tttgctgacg agaacaggga ctggtga 357
<210> 8
   <211> 1463
   <212> DNA
   <213> Artificial
<220>
   <223> 5'ccdA-KANA-3'ccdA
<400> 8
   ctgcagcgcc gcagccgccg taggcggtca cgactttgcg aagcaaagtc tagtgagtat     60
   actcaagcat tgagtggccc gccggaggca ccgccttgcg ctgcccccgt cgagccggtt     120
   ggtcaccaaa agggaggggc aggcatgaag cagcgtatta cagtgacagt tgacagcgac    180
   agctatcagt tgctcaaggc atatgatgtc aatatctccg gtctggtaag cacaaccatg    240
   cagaatgaag cccgtcgtct gcgtgccgaa cgggaacccc tatttgttta tttttctaaa    300
   tacattcaaa tatgtatccg ctcatgaatt aattcttaga aaaactcatc gagcatcaaa    360
   tgaaactgca atttattcat atcaggatta tcaataccat atttttgaaa aagccgtttc    420
   tgtaatgaag gagaaaactc accgaggcag ttccatagga tggcaagatc ctggtatcgg    480
   tctgcgattc cgactcgtcc aacatcaata caacctatta atttcccctc gtcaaaaata    540
   aggttatcaa gtgagaaatc accatgagtg acgactgaat ccggtgagaa tggcaaaagt    600
   ttatgcattt ctttccagac ttgttcaaca ggccagccat tacgctcgtc atcaaaatca    660
   ctcgcatcaa ccaaaccgtt attcattcgt gattgcgcct gagcgagacg aaatacgcga    720
   tcgctgttaa aaggacaatt acaaacagga atcgaatgca accggcgcag gaacactgcc    780
   agcgcatcaa caatattttc acctgaatca ggatattctt ctaatacctg gaatgctgtt    840
   ttcccgggga tcgcagtggt gagtaaccat gcatcatcag gagtacggat aaaatgcttg    900
   atggtcggaa gaggcataaa ttccgtcagc cagtttagtc tgaccatctc atctgtaaca    960
   tcattggcaa cgctaccttt gccatgtttc agaaacaact ctggcgcatc gggcttccca   1020
   tacaatcgat agattgtcgc acctgattgc ccgacattat cgcgagccca tttataccca   1080
   tataaatcag catccatgtt ggaatttaat cgcggcctag agcaagacgt ttcccgttga   1140
   atatggctca taacacccct tgtattactg tttatgtaag cagacagttt tattgtgcgt   1200
   attacagtga cagttgacag cgacagctat cagttgctca aggcatatga tgtcaatatc   1260
   tccggtctgg taagcacaac catgcagaat gaagcccgtc gtctgcgtgc cgaacgctgg   1320
   aaagcggaaa atcaggaagg gatggctgag gtcgcccggt ttattgaaat gaacggctct   1380
   tttgctgacg agaacaggga ctggtgagca tgcaccattc cttgcggcgg cggtgctcaa   1440
   cggcctcaac ctactacggt acc                                           1463
<210> 9
   <211> 2030
   <212> DNA
   <213> Artificial
<220>
   <223> RBS-ompA-rEPA
<400> 9
   tctagataac gagggcaaaa aatgaaaaag acagctatcg cgattgcagt ggcactggct     60
   ggtttcgcta ccgtagcgca ggccgcgaat ttggccgaag aagctttcga cctctggaac    120
   gaatgcgcca aagcctgcgt gctcgacctc aaggacggcg tgcgttccag ccgcatgagc    180
   gtcgacccgg ccatcgccga caccaacggc cagggcgtgc tgcactactc catggtcctg    240
   gagggcggca acgacgcgct caagctggcc atcgacaacg ccctcagcat caccagcgac    300
   ggcctgacca tccgcctcga aggcggcgtc gagccgaaca agccggtgcg ctacagctac    360
   acgcgccagg cgcgcggcag ttggtcgctg aactggctgg taccgatcgg ccacgagaag    420
   ccctcgaaca tcaaggtgtt catccacgaa ctgaacgccg gcaaccagct cagccacatg    480
   tcgccgatct acaccatcga gatgggcgac gagttgctgg cgaagctggc gcgcgatgcc    540
   accttcttcg tcagggcgca cgagagcaac gagatgcagc cgacgctcgc catcagccat    600
   gccggggtca gcgtggtcat ggcccagacc cagccgcgcc gggaaaagcg ctggagcgaa    660
   tgggccagcg gcaaggtgtt gtgcctgctc gacccgctgg acggggtcta caactacctc    720
   gcccagcaac gctgcaacct cgacgatacc tgggaaggca agatctaccg ggtgctcgcc    780
   ggcaacccgg cgaagcatga cctggacatc aaacccacgg tcatcagtca tcgcctgcac    840
   tttcccgagg gcggcagcct ggccgcgctg accgcgcacc aggcttgcca cctgccgctg    900
   gagactttca cccgtcatcg ccagccgcgc ggctgggaac aactggagca gtgcggctat    960
   ccggtgcagc ggctggtcgc cctctacctg gcggcgcggc tgtcgtggaa ccaggtcgac   1020
   caggtgatcc gcaacgccct ggccagcccc ggcagcggcg gcgacctggg cgaagcgatc   1080
   cgcgagcagc cggagcaggc ccgtctggcc ctgaccctgg ccgccgccga gagcgagcgc   1140
   ttcgtccggc agggcaccgg caacgacgag gccggcgcgg ccaacgccga cgtggtgagc   1200
   ctgacctgcc cggtcgccgc cggtgaatgc gcgggcccgg cggacagcgg cgacgccctg   1260
   ctggagcgca actatcccac tggcgcggag ttcctcggcg acggcggcga cgtcagcttc   1320
   agcacccgcg gcacgcagaa ctggacggtg gagcggctgc tccaggcgca ccgccaactg   1380
   gaggagcgcg gctatgtgtt cgtcggctac cacggcacct tcctcgaagc ggcgcaaagc   1440
   atcgtcttcg gcggggtgcg cgcgcgcagc caggacctcg acgcgatctg gcgcggtttc   1500
   tatatcgccg gcgatccggc gctggcctac ggctacgccc aggaccagga acccgacgca   1560
   cgcggccgga tccgcaacgg tgccctgctg cgggtctatg tgccgcgctc gagcctgccg   1620
   ggcttctacc gcaccagcct gaccctggcc gcgccggagg cggcgggcga ggtcgaacgg   1680
   ctgatcggcc atccgctgcc gctgcgcctg gacgccatca ccggccccga ggaggaaggc   1740
   gggcgcgtga ccattctcgg ctggccgctg gccgagcgca ccgtggtgat tccctcggcg   1800
   atccccaccg acccgcgcaa cgtcggcggc gacctcgacc cgtccagcat ccccgacaag   1860
   gaacaggcga tcagcgccct gccggactac gccagccagc ccggcaaacc gccgcgcgag   1920
   gacctgaagt aactgccgcg accggccggc tcccttcgca ggagccggcc ttctcggggc   1980
   ctggccatac atcaggtttt cctgatgcca gcccaatcga atatgaattc               2030